# EUROPEAN PATENT SPECIFICATION

(11) **EP 0 942 721 B1**
(45) Date of publication and mention of the grant of the patent: **22.01.2003**
(21) Application number: 97947510.0
(22) Date of filing: 12.11.1997
(51) Int. Cl.: A61K 31/425, A61K 31/535, A61K 31/55, A61K 47/10, A61K 47/12, A61K 9/48

(54) **LIQUID COMPOSITION COMPRISING AN HIV PROTEASE INHIBITOR AND A C12-C18 FATTY ACID**
FLÜSSIGE ZUSAMMENSETZUNG, HIV PROTEASE INHIBITOREN UND C12-C18 FETTSAURE ENTHALTEND
COMPOSITION LIQUIDE COMPRENANT UN INHIBITEUR DE LA VIH PROTEASE ET UN C12-C18 ACID GRAS

(30) Priority: 21.11.1996 US 754390
(43) Date of publication of application: 22.09.1999
(60) Divisional application: 02011533.3
(73) Proprietor: Abbott Laboratories, Abbott Park, Illinois 60064-3500 (US)
(72) Inventor: LIPARI, John, Racine, WI 53406 (US); AL-RAZZAK, Laman, A., Highland Park, IL 60035 (US); GHOSH, Soumojeet, Lindenhurst, IL 60046 (US); GAO, Rong, Park City, IL 60085 (US); KAUL, Dilip, Gurnee, IL 60031 (US)
(74) Representative: Modiano, Guido, Dr.-Ing.
(86) International application number: US9720794
(87) International publication number: WO98022106

(56) References cited:
- WO-A-95/07696
- WO-A-95/09614
- WO-A-95/20384
- WO-A-97/01349
- WO-A-97/20554

## Description

### Technical Field

A liquid pharmaceutical composition providing improved oral bioavailability is disclosed for compounds which are inhibitors of HIV protease. In particular, the composition is a solution which comprises (a) the HIV protease inhibitor, (b) a pharmaceutically acceptable organic solvent and , optionally, (c) a surfactant. The composition can optionally be encapsulated in either hard gelatin capsules or soft elastic capsules (SEC).

### Background of the Invention

One measure of the potential usefulness of an oral dosage form of a new pharmaceutical agent is the bioavailability observed after oral administration of the dosage form. Various factors can affect the bioavailability of a drug when administercd orally. These factors include aqueous solubility, drug absorption throughout the gastrointestinal tract, dosage strength and first pass effect. Aqueous solubility is one of the most important of these factors. When a drug has poor aqueous solubility, attempts are often made to identify salts or other derivatives of the drug which have improved aqueous solubility. When a salt or other derivative of the drug is identified which has good aqueous solubility, it is generally accepted that an aqueous solution formulation of this salt or derivative will provide the optimum oral bioavailability. The bioavailability of the oral solution formulation of a drug is then generally used as the standard or ideal bioavailability against which other oral dosage forms are measured.

For a variety of reasons, such as patient compliance and taste masking, a solid dosage form, such as capsules, is usually preferred over a liquid dosage form. However, oral solid dosage forms of a drug generally provide a lower bioavailabiiity than oral solutions of the drug. One goal of the development of a suitable capsule dosage form is to obtain a bioavailabiiity of the drug that is as close as possible to the ideal bioavailability demonstrated by the oral solution formulation of the drug.

It has recently been determined that HIV protease inhibiting compounds are useful for inhibiting HIV protease in vitro and in vivo, are useful for inhibiting HIV (human immunodeficiency virus) infections and are useful for treating AIDS (acquired immunodeficiency syndrome). HIV protease inhibiting compounds typically are characterized by having poor oral bioavailability and there is a continuing need for the development of improved oral dosage forms for HIV protease inhibitors which have suitable oral bioavailability, stability and side effects profiles.

Known from WO 95/09614 are pharmaceutical compositions for improving oral bioavailability of an HIV protease inhibitor wherein such compositions comprise an HIV protease inhibitor which is preferably ritonavir, an organic solvent and a pharmaceutically acceptable acid. The acid can be *inter alia* undecenoic acid. In a preferred composition, the amount of ritonavir is up to 40% w/w and the amount of the acid is up to 2 molar equivalents based on the amount of ritonavir.

Examples of HIV protease inhibiting compounds include N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)-piperazinyl))-pentaneamide (i.e., indinavir) and related compounds, disclosed in EP-A-541168, published May 12. 1993, and U.S. Patent No. 5,413,999, issued May 9, 1995; N-tert-butyl-decahydro-2-[2(R)-hydroxy-4-phenyl-3(S)-[[N-(2-quinolylcarbonyl)-L-asparaginyl]amino]butyl]-(4aS,8aS)-isoquinoline-3(S)-carboxamide (i.e., saquinavir) and related compounds, disclosed in U.S. Patent No. 5,196,438, issued March 23, 1993; 5(S)-Boc-amino-4(S)-hydroxy-6-phenyl-2(R)-phenylmethy(hexanoyl-(L)-Val-(L)-Phe-morpholin-4-ylamide and related compounds, disclosed in EP-A-532466, published March 17, 1993; 1-Naphthoxyacetyl-beta-methylthio-Ala-(2S,3S)-3-amino-2-hydroxy-4-butanoyl-1,3-thiazolidine-4-t-butylamide (i.e., 1-Naphthoxyacetyl-Mta-(2S,3S)-AHPBA-Thz-NH-tBu), 5-isoquinolinoxyacetyl-beta-methylthio-Ala-(2S,3S)-3-amino-2-hydroxy-4-butanoyl-1,3-thiazolidine-4-t-butylamide (i.e., iQoa-Mta-Apns-Thz-NHtBu) and related compounds, disclosed in EP-A-490667, published June 17,1992 and Chem. Pharm. Bull. 40 (8) 2251 (1992),; [1S-[1R*(R*),2S*]}-N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide (i.e., SC-52151) and related compounds, disclosed in PCT Patent Application No. WO92/08701, published May 29, 1992 and PCT Patent Application No. WO93/23368, published November 25, 1993; 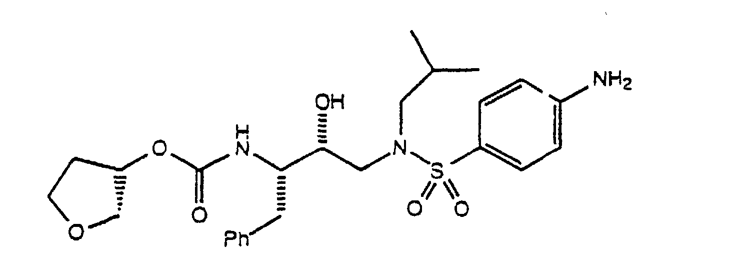 (i.e., VX-478) and related compounds, disclosed in PCT Patent Application No. WO94/05639, published March 17, 1994; 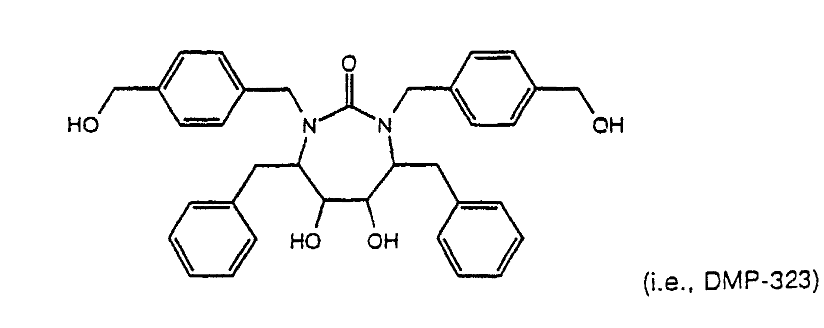 or 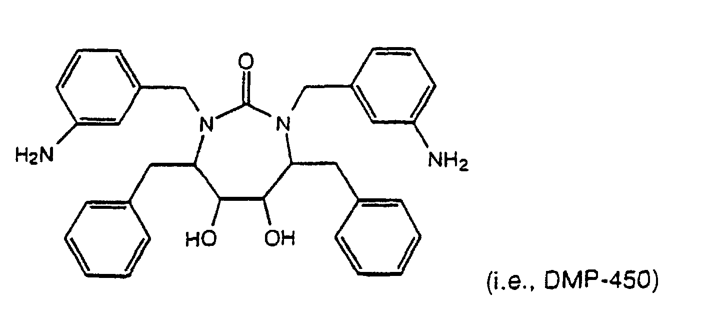 and related compounds, disclosed in PCT Patent Application No. WO93/07128, published April 15, 1993; 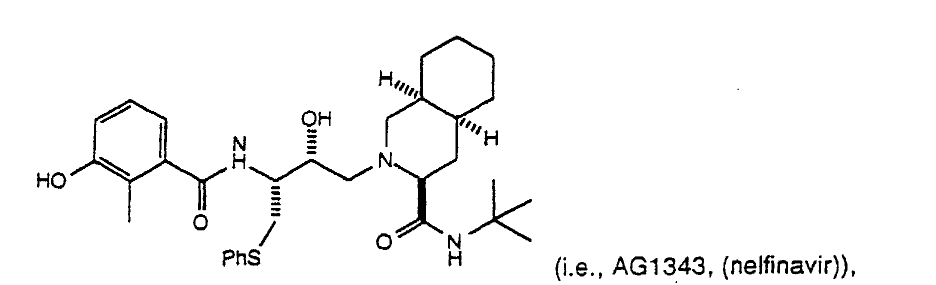 disclosed in PCT Patent Application No. WO95/09843, published April 13, 1995 and U.S. Patent No. 5,484,926. issued January 16, 1996; 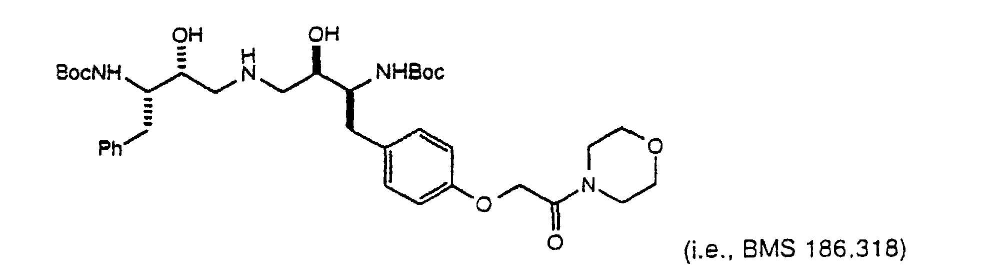 disclosed in EP-A-580402, published January 26, 1994; 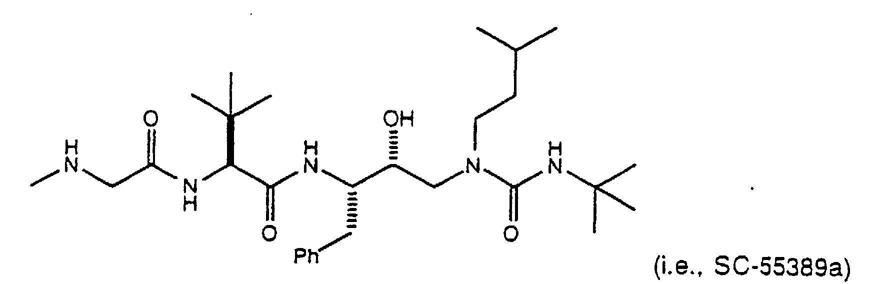 and related compounds disclosed in PCT Patent Application No. WO 9506061. published March 2, 1995 and at 2nd National Conference on Human Retroviruses and Related Infections, (Washington, D.C.. Jan. 29 - Feb. 2, 1995), Session 88; and 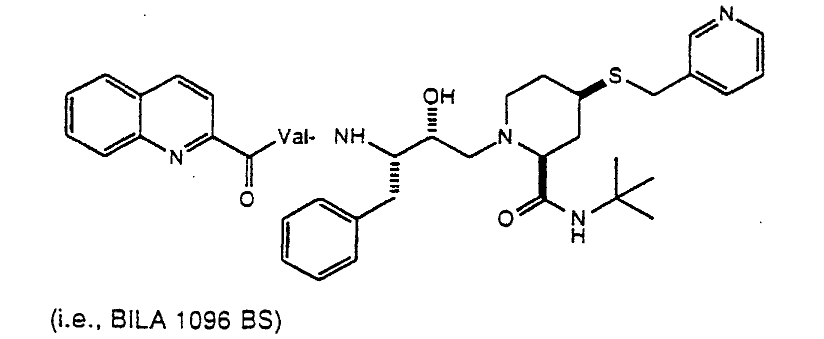 and related compounds disclosed in EP-A-560268, published September 15, 1993; and 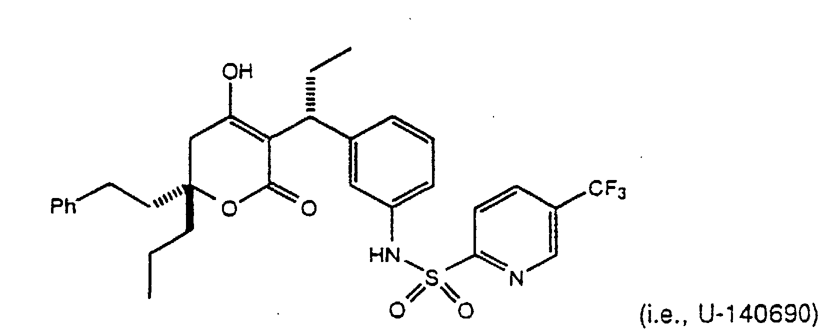 and related compounds disclosed in PCT Patent Application No. WO 9530670, published November 16, 1995;
or a pharmaceutically acceptable salt of any of the above.

Other examples of HIV protease inhibiting compounds include compounds of the formula **I**: 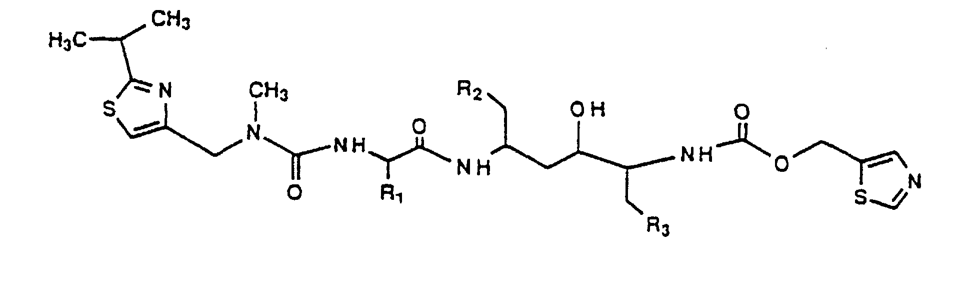 wherein R₁ is lower alkyl and R₂ and R₃ are phenyl and related compounds or a pharmaceutically acceptable salt thereof. disclosed in PCT Patent Application No. WO94/14436, published July 7, 1994 and U.S. Patent No. 5,541,206, issued July 30, 1996. The compounds of formula **I** are useful to inhibit HIV infections and, thus, are useful for the treatment of AIDS.

In particular, the compound of formula **II**. has been found to be especially effective as an inhibitor of HIV protease.

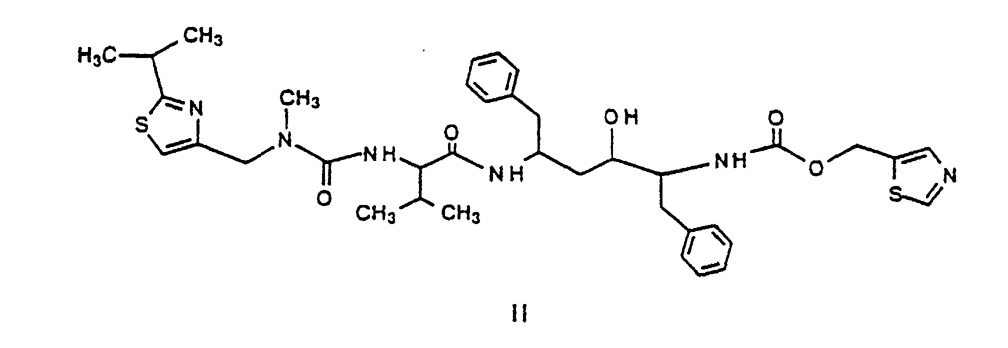

The most preferred compound of formula **II** is (2S,3S,5S)-5-(N-(N-((N-Methyl-N-((2-isopropyl-4-thiazolyl)methyl)-amino)carbonyl)valinyl)amino)-2-(N-((5-thiazolyl)methoxycarbonyl)amino)-1,6-diphenyl-3-hydroxyhexane (ritonavir; compound **III**) or a pharmaceutically acceptable salt thereof.

Other examples of HIV protease inhibiting compounds also include compounds of the formula **IV**: 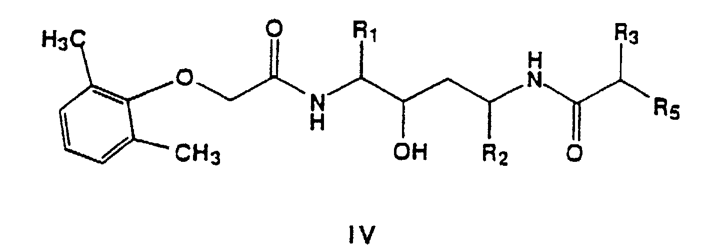 wherein R₁ is benzyl, R₂ is benzyl or loweralkyl, R₃ is loweralkyl and R₅ is 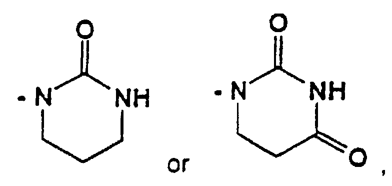 and related compounds or a pharmaceutically acceptable salt thereof, disclosed in

International Patent Application No. WO97/21685, published June 19, 1997.

A preferred compound is the compound of formula **IV** wherein R₁ and R₂ are benzyl, R₃ is isopropyl and R₅ is

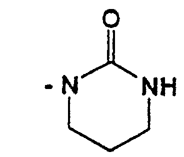

A most preferred compound of the formula **IV** is (2S. 3S, 5S)-2-(2,6-Dimethylphenoxyacetyl) amino-3-hydroxy-5-[2S-(1-tetrahydro-pyrimid-2-onyl)-3-methyl butanoyl] amino-1,6-diphenylhexane (compound **V**) or a pharmaceutically acceptable salt thereof. The preparation of compound V is disclosed in International Patent Application No. WO97/21685, published June 19, 1997.

Compound **III** has an aqueous solubility of approximately 6 micrograms per milliliter at pH >2. This is considered to be extremely poor aqueous solubility and, therefore, compound **III** in the free base form would be expected to provide very low oral bioavailabitity. In fact, the free base form of compound **III**, administered as an unformulated solid in a capsule dosage form, is characterized by a bioavailability of less than 2% following a 5 mg/kg oral dose in dogs.

Acid addition salts of compound **III** (for example, bis-hydrochloride, bis-tosylate, bis-methane sulfonate and the like) have aqueous solubilities of <0.1 milligrams/milliliter. This is only a slight improvement over the solubility of the free base. This low aqueous solubility would not make practical the administration of therapeutic amounts of an acid addition salt of compound **III** as an aqueous solution. Furthermore, in view of this low aqueous solubility, it is not surprising that the bis-tosylate of compound **III**, administered as an unformulated solid in a capsule dosage form, is characterized by a bioavailability of less than 2% following a 5 mg/kg oral dose in dogs.

In order to have a suitable oral dosage form of compound **III**, the oral bioavailability of compound **III** should be at least 20%. Preferably, the oral bioavailability of compound **III** from the dosage form should be greater than about 40% and, more preferably, greater than about 50%.

While some drugs would be expected to have good solubility in organic solvents, it would not necessarily follow that oral administration of such a solution would give good bioavailability for the drug. It has been found that compound III has good solubility in pharmaceutically acceptable organic solvents and that the solubility in such solvents is enhanced in the presence of a pharmaceutically acceptable long chain fatty acid. Administration of the solution as an encapsulated dosage form (soft elastic capsules or hard gelatin capsules) provides an oral bioavailability of as high as about 60% or more.

### Disclosure of the Invention

In accordance with the present invention, there is a pharmaceutical composition which is a solution comprising
(a) an HIV protease inhibiting compound of the formula **II** or a pharmaceutically acceptable salt thereof, preferably a compound of of the formula **III** or a pharmaceutically acceptable salt thereof, or a combination of a compound of the formula **II** or a pharmaceutically acceptable salt thereof and another HIV protease inhibitor or a pharmaceutically acceptable salt thereof (preferably, the compound of the formula **IV** or saquinavir or indinavir or nelfinavir or VX-478), or, more preferably, a combination of a compound of the formula III and another HIV protease inhibitor (preferably, the compound of the formula **V** or saquinavir or indinavir or nelfinavir or VX-478), or, most preferably, a combination of a compound of formula **III** and a compound of formula V, in the total amount of from 1 % to 50% (preferably, from 1% to 40%; more preferably, from 10 % to 40%; most preferably, from 15% to 40%) by weight of the total solution,
(b) a pharmaceutically acceptable organic solvent which comprises (i) a pharmaceutically acceptable long chain fatty acid in the amount of from 30% to 99% (preferably, from 30% to 70%; more preferably, from . 40% to 60%) by weight of the total solution or (ii) a mixture of (1) a pharmaceutically acceptable long chain fatty add in the amount of from 30% to 99% (preferably, from 30% to 70%; more preferably, from 40% to 60%) by weight of the total solution and (2) a pharmaceutically acceptable alcohol in the amount of from 0% to 15% (preferably, from 6% to 12%) by weight of the total solution and
(c) a pharmaceutically acceptable surfactant in the amount of from 0% to 40% (preferably, from 2% to 20% and most preferably, from 5% to 15%) by weight of the total solution. In a preferred embodiment of the invention, the solution is encapsulated in a soft elastic gelatin capsule (SEC) or a hard gelatin capsule.

The solution composition of the invention can also comprise an antioxidant (for example, ascorbic acid, BHA (butylated hydroxyanisole), BHT (butylated hydroxytoluene), vitamin E, vitamin E PEG 1000 succinate and the like) for chemical stability.

The compositions of this invention (solution or encapsulated solution) provide improved oral bioavailability for HFV protease inhibitors. In particular, the compositions of this invention (solution or encapsulated solution) provide improved oral bioavailability for compound **III** when compared to non-formulated compound **III** (base) or non-formulated compound **III** (acid addition salt).

The term "pharmaceutically acceptable long chain fatty acid" as used herein refers to saturated, mono-unsaturated or di-unsaturated C₁₂ to C₁₈ carboxylic acids which are liquids at room temperature. Preferred long chain fatty acids are mono-unsaturated C₁₆-C₁₈ carboxylic acids which are liquids at room temperature. A most preferred long chain fatty acid is oleic acid.

The term "pharmaceutically acceptable alcohol" as used herein refers to alcohols which are liquids at about room temperature, approximately 20°C, for example, ethanol, propylene glycol, 2-(2-ethoxyethoxy)ethanol (Transcutol®. Gattefosse, Westwood. NJ 07675), benzyl alcohol, glycerol, polyethylene glycol 200, polyethylene glycol 300, polyethylene glycol 400 and the like. A preferred pharmaceutically acceptable alcohol is ethanol or propylene glycol or a mixture thereof.

The term "pharmaceutically acceptable surfactant" as used herein refers to a pharmaceutically acceptable non-ionic surfactant for example, polyoxyethylene castor oil derivatives (for example, polyoxyethyleneglyceroltriricinoleate or polyoxyl 35 castor oil (Cremophor®EL, BASF Corp.) or polyoxyethyleneglycerol oxystearate (Cremophor®RH 40 (polyethyleneglycol 40 hydrogenated castor oil)) or Cremophor®RH 60 (polyethyleneglycol 60 hydrogenated castor oil), BASF Corp. and the like) or block copolymers of ethylene oxide and propylene oxide, also known as polyoxyethylene polyoxypropylene block copolymers or polyoxyethylenepolypropylene glycol, such as Poloxamer®124, Poloxamer®188, Poloxamer®237, Poloxamer®388, Poloxamer®407 and the like, (BASF Wyandotte Corp.) or a mono fatty acid ester of polyoxyethylene (20) sorbitan (for example, polyoxyethylene (20) sorbitan monooleate (Tween® 80). polyoxyethylene (20) sorbitan monostearate (Tween® 60), polyoxyethylene (20) sorbitan monopalmitate (Tween® 40), polyoxyethylene (20) sorbitan monolaurate (Tween® 20) and the like) and the like) or a sorbitan fatty acid ester (including sorbitan laurate, sorbitan oleate, sorbitan palmitate, sorbitan stearate and the like). A preferred pharmaceutically acceptable surfactant is polyoxyl 35 castor oil (Cremophor®EL, BASF Corp.), polyoxyethylene (20) sorbitan monolaurate (Tween® 20), polyoxyethylene (20) sorbitan monooleate (Tween® 80) or a sorbitan fatty acid ester, for example sorbitan oleate. A most preferred pharmaceutically acceptable surfactant is polyoxyl 35 castor oil (Cremophor®EL, BASF Corp.).

Preferably, the pharmaceutically acceptable organic solvent comprises from 50% to 99% by weight of the total solution. More preferably. the pharmaceutically acceptable organic solvent or mixture of pharmaceutically acceptable organic solvents comprises from 50% to 75% by weight of the total solution.

Preferred pharmaceutically acceptable solvents comprise (1) a pharmaceutically acceptable long chain fatty acid in the amount of from 40% to 70% by weight of the total solution and (2) ethanol or propylene glycol in the amount of from 1 % to 15% by weight of the total solution or a mixture of ethanol and propylene glycol in the amount of from 1% to 15% by weight of the total solution. More preferred pharmaceutically acceptable solvents comprise (1) a pharmaceutically acceptable long chain fatty acid in the amount of from 40% to 70% by weight of the total solution and (2) ethanol in the amount of from 10% to 12% by weight of the total solution or propylene glycol in the amount of from 5% to 10% by weight of the total solution or a mixture of ethanol and propylene glycol in the amount of from 5% to 15% by weight of the total solution. Even more preferred pharmaceutically acceptable solvents comprise (1) oleic acid in the amount of from 40% to 70% by weight of the total solution and (2) ethanol in the amount of from 10% to 12% by weight of the total solution or propylene glycol in the amount of from 5% to 10% by weight of the total solution or a mixture of ethanol and propylene glycol in the amount of from 10% to 15% by weight of the total solution.

In one embodiment of the invention, a more preferred composition of the invention is a solution comprising
(a) ritonavir in the amount of from 1% to 30% (preferably, from 5% to 25%) by weight of the total solution,
(b) a pharmaceutically acceptable organic solvent which comprises
   (i) a pharmaceutically acceptable long chain fatty acid in the amount of from 30% to 99% (preferably, from 30% to 70%; more preferably, from 40% to 60%) by weight of the total solution or (ii) a mixture of (1) a pharmaceutically acceptable long chain fatty acid in the amount of from 30% to 99% (preferably, from 30% to 70%; more preferably, from 40% to 60%) by weight of the total solution and (2) a pharmaceutically acceptable alcohol in the amount of from 0% to 15% (preferably, from 6% to 12%) by weight of the total solution and
(c) a pharmaceutically acceptable surfactant in the amount of from 0% to 20% (preferably, from 5% to 10%) by weight of the total solution. In a more preferred embodiment of the invention, the solution is encapsulated in a soft elastic gelatin capsule (SEC) or a hard gelatin capsule.

An even more preferred composition of the invention is a solution comprising
(a) ritonavir in the amount of from 1% to 30% (preferably, from 5% to 25%) by weight of the total solution,
(b) a pharmaceutically acceptable organic solvent which comprises (i) oleic acid in the amount of from 30% to 99% (preferably, from 30% to 70%; more preferably, from 40% to 60%) by weight of the total solution or (ii) a mixture of (1) oleic acid in the amount of from 30% to 99% (preferably, from 30% to 70%; more preferably, from 40% to 60%) by weight of the total solution and (2) ethanol in the amount of from 0% to 12% (preferably, from 10% to 12%) by weight of the total solution or propylene glycol in the amount of from 0% to 10% (preferably, from 5% to 10%) by weight of the total solution or a mixture thereof in the amount of from 0% to 15% (preferably, from 10% to 15%) by weight of the total solution and
(c) polyoxyl 35 castor oil in the amount of from 0% to 20% (preferably, from 5% to 10%) by weight of the total solution. In an even more preferred embodiment of the invention, the solution is encapsulated in a soft elastic gelatin capsule (SEC) or a hard gelatin capsule.

A most preferred composition of the invention is a solution comprising
(a) ritonavir in the amount of about 20% by weight of the total solution,
(b) a pharmaceutically acceptable organic solvent which comprises a mixture of (1) oleic acid in the amount of from 62% to 64%by weight of the total solution and (2) ethanol in the amount of from 10% to 12%, preferably, about 12%, by weight of the total solution and
(c) polyoxyl 35 castor oil in the amount of about 6% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of about 0.05% by weight of the total solution.

Another most preferred composition of the invention is a solutoin comprising
(a) ritonavir in the amount of about 20% by weight of the total solution,
(b) a pharmaceutically acceptable organic solvent which comprises a mixture of (1) oleic acid in the amount of about 65% by weight of the total solution and (2) ethanol in the amount of about 10% by weight of the total solution and
(c) polyoxyl 35 castor oil in the amount of about 5% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from 0.01% to 0.08% by weight of the total solution (preferably, from 0.01 % to 0.05% by weight of the total solution).

Another most preferred composition of the invention is a solution comprising
(a) ritonavir in the amount of about 20% by weight of the total solution,
(b) a pharmaceutically acceptable organic solvent which comprises a mixture of (1) oleic acid in the amount of about 60% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoiuene)) in the amount of from 0.01% to 0.08% by weight of the total solution (preferably, from 0.01% to 0.05% by weight of the total solution).

Another most preferred composition of the invention is a solution comprising
(a) ritonavir in the amount of about 20% by weight of the total solution,
(b) a pharmaceutically acceptable organic solvent which comprises
   a mixture of (1) oleic acid in the amount of about 70% by weight of the total solution and (2) a mixture of ethanol in the amount of about 5% by weight of the total solution and propylene glycol in the amount of about 5% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from 0.01% to 0.08% by weight of the total solution (preferably, from 0.01 % to 0.05% by weight of the total solution).

In yet another embodiment of the invention, a more preferred composition of the invention is a solution comprising
(a) a mixture of ritonavir in the amount of from 1% to 30% (preferably, from 5% to 25%) by weight of the total solution and another HIV protease inhibitor in the amount of from 1% to 50% (preferably, from 5% to 40%) by weight of the total solution,
(b) a pharmaceutically acceptable organic solvent which comprises (i) a pharmaceutically acceptable long chain fatty acid in the amount of from 30% to 98% (preferably, from 30% to 70%; more preferably, from 40% to 65%) by weight of the total solution or (ii) a mixture of (1) a pharmaceutically acceptable long chain tarty acid in the amount of from 30% to 98% (preferably, from 30% to 70%; more preferably, from 40% to 65%) by weight of the total solution and
(2) a pharmaceutically acceptable alcohol in the amount of from 0% to 15% (preferably, from 6% to 12%) by weight of the total solution and
(c) a pharmaceutically acceptable surfactant in the amount of from 0% to 20% (preferably, from 5% to 10%) by weight of the total solution. In a more preferred embodiment of the invention, the solution is encapsulated in a soft elastic gelatin capsule (SEC) or a hard gelatin capsule.

An even more preferred composition of the invention is a solution comprising
(a) a mixture of ritonavir in the amount of from 1% to 30% (preferably, from 5% to 25%) by weight of the total solution and another HIV protease inhibitor in the amount of from 1% to 50% (preferably, from 5% to 40%) by weight of the total solution,
(b) a pharmaceutically acceptable organic solvent which comprises (i) oleic acid in the amount of from 30% to 98% (preferably, from 30% to 70%; more preferably, from 40% to 65%) by weight of the total solution or (ii) a mixture of (1) oleic acid in the amount of from 30% to 98% (preferably, from 30% to 70%; more preferably, from 40% to 65%) by weight of the total solution and (2) ethanol in the amount of from 0% to . 12% (preferably, from 10% to 12%) by weight of the total solution or propylene glycol in the amount of from 0% to 10% (preferably, from 5% to 10%) by weight of the total solution or a mixture thereof in the amount of from 0% to 15% (preferably, from 10% to 15%) by weight of the total solution and
(c) polyoxyl 35 castor oil in the amount of from 0% to 20% (preferably, from 5% to 10%) by weight of the total solution. In an even more preferred embodiment of the invention, the solution is encapsulated in a soft elastic gelatin capsule (SEC) or a hard gelatin capsule.

A most preferred composition of the invention is a solution comprising
(a) a mixture of ritonavir in the amount of from 1% to 30% (preferably, from 5% to 25%) by weight of the total solution and (2S, 3S, 5S)-2-(2,6-dimethylphenoxyacetyl) amino-3-hydroxy-5-[2S-(1-tetrahydropyrimid-2-onyl)-3-methyl butanoyl] amino-1,6-diphenylhexane in the amount of from 1% to 50% (preferably, from 5% to 40%) by weight of the total solution,
(b) a pharmaceutically acceptable organic solvent which comprises a mixture of (1) oleic acid in the amount of from 30% to about 88% (preferably, from 40% to 65%) by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most preferred composition of the invention is a solution comprising
(a) a mixture of ritonavir in the amount of from 1 % to 30% (preferably, from 5% to 25%) by weight of the total solution and (2S, 3S, 5S)-2-(2,6-dimethylphenoxyacetyl) amino-3-hydroxy-5-[2S-(1-tetrahydropyrimid-2-onyl)-3-methyl butanoyl] amino-1,6-diphenylhexane in the amount of from 1% to 50% (preferably, from 5% to 40%) by weight of the total solution,
(b) a pharmaceutically acceptable organic solvent which comprises a mixture of (1) oleic acid in the amount of from 30% to 88% (preferably, from 40% to 65%) by weight of the total solution and (2) propylene glycol in the amount of from 5% to 10% (preferably, from 6% to 8%, by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

A most highly preferred composition of the invention is a solution comprising
(a) a mixture of ritonavir in the amount of about 5% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 30% by weight of the total solution,
(b) a pharmaceutically acceptable organic solvent which comprises a mixture of (1) oleic acid in the amount of about 45% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.03% to about 0.05% by weight of the total solution).

Another most highly preferred composition of the invention is a solution comprising
(a) a mixture of ritonavir in the amount of about 15% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 15% by weight of the total solution,
(b) a pharmaceutically acceptable organic solvent which comprises a mixture of (1) oleic acid in the amount of about 50% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.03% to about 0.05% by weight of the total solution).

Another most highly preferred composition of the invention is a solution comprising
(a) a mixture of ritonavir in the amount of about 15% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 5% by weight of the total solution,
(b) a pharmaceutically acceptable organic solvent which comprises a mixture of (1) oleic acid in the amount of about 60% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.03% to about 0.05% by weight of the total solution).

Another most highly preferred composition of the invention is a solution comprising
(a) a mixture of ritonavir in the amount of about 10% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 20% by weight of the total solution,
(b) a pharmaceutically acceptable organic solvent which comprises a mixture of (1) oleic acid in the amount of about 50% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.03% to about 0.05% by weight of the total solution).

Another most highly preferred composition of the invention is a solution comprising
(a) a mixture of ritonavir in the amount of about 13% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 17% by weight of the total solution,
(b) a pharmaceutically acceptable organic solvent which comprises a mixture of (1) oleic acid in the amount of about 50% by weight of the total solution and (2) ethanol in the amount of about 10% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.03% to about 0.05% by weight of the total solution).

Another most highly preferred composition of the invention is a solution comprising
(a) a mixture of ritonavir in the amount of about 6.0% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 24% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention comprises a solution of (a) a mixture of ritonavir in the amount of about 5% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 25% by weight of the total solution and (b) polyoxyl 35 castor oil in the amount of about 10% by weight of the total solution, in a pharmaceutically acceptable organic solvent which comprises a mixture of (1) oleic acid in the amount of about 52.5% by weight of the total solution and (2) propylene glycol in the amount of about 7.5% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention is a solution comprising
(a) a mixture of ritonavir in the amount of about 8% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 24% by weight of the total solution,
(b) a pharmaceutically acceptable organic solvent which comprises a mixture of (1) oleic acid in the amount of about 50.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition.of the invention is a solution comprising
(a) a mixture of ritonavir in the amount of about 8.25% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 22% by weight of the total solution,
(b) a pharmaceutically acceptable organic solvent which comprises a mixture of (1) oleic acid in the amount of about 52.25% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention is a solution comprising
(a) a mixture of ritonavir in the amount of about 5% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 30% by weight of the total solution,
(b) a pharmaceutically acceptable organic solvent which comprises a mixture of (1) oleic acid in the amount of about 47.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention is a solution comprising
(a) a mixture of ritonavir in the amount of about 13% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 17% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention is a solution comprising
(a) a mixture of ritonavir in the amount of about 15% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 15% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

Another most highly preferred composition of the invention is a solution comprising
(a) a mixture of ritonavir in the amount of about 10% by weight of the total solution and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane in the amount of about 20% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution. In a most preferred embodiment of the invention, the solution is encapsulated in a soft elastic gelatin capsule (SEC) or a hard gelatin capsule and the solution also comprises an antioxidant (preferably, BHT (butylated hydroxytoluene)) in the amount of from about 0.01% to about 0.08% by weight of the total solution (preferably, from about 0.01% to about 0.05% by weight of the total solution).

In the compositions of the invention, preferred combinations of HIV protease inhibitors include ritonavir and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane, ritonavir and indinavir, ritonavir and saquinavir, ritonavir and nelfinavir, and ritonavir and VX-478.

In the compositions of the invention which comprise a mixture cf ritonavir and (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane, the ratio (w/w) of ritonavir to (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenyfhexane ranges from 1:16 to 5:1 (preferably, from 1:8 to 3:1).

The compounds of formula I, II and IV contain two or more asymmetric carbon atoms and thus can exist as pure diastereomers, mixtures of diastereomers, diastereomeric racemates or mixtures of diastereomeric racemates. The present invention is intended to include within its scope all of the isomeric forms. The terms "R" and "S" configuration as used herein are as defined by IUPAC 1974 Recommendations for Section E, Fundamental Stereochemistry, Pure Appl. Chem. (1976) 45. 13-30.

The preferred isomer of the compound of formula **II** is (2S,3S,5S)-5-(N-(N-((N-Methyl-N-((2-isopropyl-4-thiazolyl)methyl)-amino)carbonyl)-valinyl)amino)-2-(N-((5-thiazolyl)methoxycarbonyl)amino)-1,6-diphenyl-3-hydroxyhexane (compound **III**). The preferred isomer of the compound of formula **IV** is (2S, 3S, 5S)-2-(2,6-dimethylphenoxyacetyl) amino-3-hydroxy-5-[2S-(1-tetrahydro-pyrimid-2-onyl)-3-methyl butanoyl] amino-1,6-diphenylhexane (compound **V**).

The term "lower alkyl" as used herein refers to straight or branched chain alkyl radicals containing from 1 to 6 carbon atoms including, but not limited to, methyl, ethyl, n-propyl, iso-propyl, n-butyl, iso-butyl, sec-butyl, n-pentyl, 1-methylbutyl, 2,2-dimethylbutyl, 2-methylpentyl, 2,2-dimethylpropyl, n-hexyl and the like.

The HIV protease inhibiting compounds can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The composition and preparation of the soft elastic gelatin capsule itself is well known in the art. The composition of a soft elastic gelatin capsule typically comprises from about 30% to about 50% by weight of gelatin NF, from about 10% to about 40% by weight of a plasticizer or a blend of plasticizers and from about 25% to about 40% by weight of water. Plasticizers useful in the preparation of soft elastic gelatin capsules are glycerin, sorbitol or sorbitol derivatives (for example, sorbitol-special and the like) or propylene glycol and the like; or combinations thereof.

The soft elastic gelatin capsule material can also comprise additives such as preservatives, opacifiers, pigments, dyes or flavors and the like.

Various methods can be used for manufacturing and filling the soft elastic gelatin capsules, for example, a seamless capsule method, a rotary method (developed by Scherer) or a method using a Liner machine or an Accogel machine and the like. Also various manufacturing machines can be used for manufacturing the capsules.

Typically, the soft elastic gelatin capsule is prepared by (1) preparing the gel mass, (2) encapsulating the fill material (forming, filling and sealing the capsule) and (3) softgel drying.

During gel mass preparation, the ingredients comprising the gel mass (typically, gelatin, water and plasticizer) are mixed to form a uniform fluff. After blending, the fluff gel mass is melted, preferably, under vacuum, and the melted gel mass is transferred to heated receivers. Colorants or other additives can be added to the melted gel mass, which is then blended until uniform.

In one method a rotary die encapsulation apparatus is then used to encapsulate the liquid capsule fill. In general, in this method two gel ribbons are fed between two rotating dies. The dies contain paired pockets which form the shape of the softgel and provide the sealing mechanism. At the moment the two die half pockets line up, the fill material is injected through an encapsulation wedge in between the gel ribbons. The softgel is formed and sealed as a result of pressure between the dies and heat applied by the encapsulation wedge.

Finally, the filled softgels are dried. In one method the filled softgels are first placed in a rotary drier in a low humidity, forced air environment. A final step in the drying process involves discharging the filled softgels from the rotary drier and placing them in a monolayer on shallow drying trays, over which is circulated low humidity air of less than 50% relative humidity. The drying process is stopped by transferring the softgels into deep holding trays.

Preferred soft elastic gelatin capsules are manufactured by R.P. Scherer Corp.

Hard gelatin capsules can be purchased from Capsugel, Greenwood, SC and other suppliers. Capsules are filled manually or by capsule filling machine. The target filling volume/weight depends on the potency of the filling solution in combination with the desired dosage strength.

In general, the compositions of this invention can be prepared in the following manner. The pharmaceutically acceptable long chain fatty acid and the pharmaceutically acceptable alcohol are mixed at room temperature, along with the antioxidant. The HIV protease inhibitor, or mixture of HIV protease inhibitors, is added and stirred until dissolved. The pharmaceutically acceptable surfactant is added with mixing. The appropriate volume of the resulting mixture needed to provide the desired dose of the HIV protease inhibiting compound(s) is filled into hard gelatin capsules or soft elastic gelatin capsules.

The following examples will serve to further illustrate the invention.

### Example 1 (non-formulated capsule)

An amount of compound **III** (free base) equivalent to a 5 mg/kg dose was placed in hard gelatin capsules (gray, size 0). These capsules were administered to fasted dogs with 10 ml of water.

### Example 2 (Capsule)

An amount of compound **III** (free base) equivalent to a 5 mg/kg dose was placed in hard gelatin capsules (gray, size 0). These capsules were administered to non-fasted dogs with ten milliliter of water.

### Example 3 (Capsule)

An amount of the bis-tosylate salt of compound **III** equivalent to a 5 mg/kg dose of compound **III** (base equivalent) was filled into hard gelatin capsules (gray, size 0). These capsules were administered to fed dogs with ten milliliter of water.

### Example 4 (capsule)

| Component | % By Weight |
|---|---|
| ritonavir (free base) | 20 |
| Ethanol (USP, 200 proof) | 10 |
| Oleic acid, 6321, NF | 69.99 |
| Butylated hydroxy toluene (BHT), NF | 0.01 |

### Example 5 (capsule)

| Component | % By Weight |
|---|---|
| ritonavir (free base) | 20 |
| Ethanol (USP, 200 proof) | 10 |
| Tween® 80 (NF) | 5 |
| Oleic acid, 6321, NF | 64.99 |
| Butylated hydroxy toluene (BHT), NF | 0.01 |

### Example 6 (capsule)

| Component | % By Weight |
|---|---|
| ritonavir (free base) | 20 |
| Ethanol (USP, 200 proof) | 10 |
| Tween® 20 (NF) | 5 |
| Oleic acid, 6321, NF | 64.99 |
| Butylated hydroxy toluene (BHT), NF | 0.01 |

### Example 7 (capsule or SEC)

| Component | % By Weight |
|---|---|
| ritonavir (free base) | 20 |
| Ethanol (USP, 200 proof) | 10 |
| polyoxyl 35 castor oil (Cremophor® EL) | 5 |
| Oleic acid, 6321, NF | 64.99 |
| Butylated hydroxy toluene (BHT), NF | 0.01 |

The mixing tank was purged with nitrogen. Oleic acid (649.9 g) and ethanol (100g) were mixed in the tank. This solution was warmed to about 33°C (28-37°C) and maintained at that temperature. The butylated hydroxytoluene (0.1 g) was charged into the tank and mixed until the solution was clear. The ritonavir (200 g) was slowly charged into the tank and mixed until the solution was clear. The polyoxyl 35 castor oil (50 g) was added to the tank and mixed. Heating was discontinued and the solution allowed to cool to amibient temperature (20-30°C). The resulting solution was filled into soft elastic capsules (0.5 g of solution/SEC) to provide a dosage of 100 mg of ritonavir/SEC or 1.0 g of solution/SEC to provide a dosage of 200 mg of ritonavir/SEC.

### Example 8 (capsule)

| Component | % By Weight |
|---|---|
| ritonavir (free base) | 20 |
| Ethanol (USP, 200 proof) | 10 |
| polyoxyl 35 castor oil (Cremophor® EL) | 4.5 |
| Sorbitan monooleate | 0.5 |
| Oleic acid, 6321, NF | 64.99 |
| Butylated hydroxy toluene (BHT), NF | 0.01 |

### Example 9 (capsule or SEC)

| Component | % By Weight |
|---|---|
| ritonavir (free base) | 20 |
| Ethanol (USP, 200 proof) | 10 |
| polyoxyl 35 castor oil (Cremophor® EL) | 10 |
| Oleic acid, 6321, NF | 59.99 |
| Butylated hydroxy toluene (BHT), NF | 0.01 |

The mixing tank was purged with nitrogen. Oleic acid (599.9 g) and ethanol (100g) were mixed in the tank. This solution was warmed to about 33°C (28-37°C) and maintained at that temperature. The butylated hydroxytoluene (0.1 g) was charged into the tank and mixed until the solution was clear. The ritonavir (200 g) was slowly charged into the tank and mixed until the solution was clear. The polyoxyl 35 castor oil (100 g)was added to the tank and mixed. Heating was discontinued and the solution allowed to cool to amibient temperature (20-30°C). The resulting solution was filled into soft elastic capsules (0.5 g of solution/SEC) to provide a dosage of 100 mg of ritonavir/SEC or 1.0 g of solution/SEC to provide a dosage of 200 mg of ritonavir/SEC.

### Example 10 (SEC)

| Component | % By Weight |
|---|---|
| ritonavir (free base) | 20 |
| Ethanol (USP, 200 proof) | 12 |
| polyoxyl 35 castor oil (Cremophor® EL) | 6 |
| Oleic acid, 6321, NF | 61.95 |
| Butylated hydroxy toluene (BHT), NF | 0.05 |

The mixing tank was purged with nitrogen. Ethanol (118 g) was weighed out and blanketed with nitrogen. Ethanol (2 g) and butylated hydroxytoluene (0.5 g) were charged into a second mixing tank and mixed until uniform under a blanket of nitrogen. The main mixing tank was set for a temperature of 28°C (range 23-33°C). Oleic acid (614.5 g) was charged into the main mixing tank and mixing began. Ritonavir (200 g) was charged into the main mixing tank while mixing and mixed until uniform. The ethanol and and ethanol/butylated hydroxytoluene mixture were charged into the main mixing tank and mixed until clear. The polyoxyl 35 castor oil (60 g) was charged into the main mixing tank. Oleic acid (5 g) was charged into the main mixing tank and mixed until clear. The resulting solution was discharged through a 70 mesh or finer filter for storage at 2-8°C under nitrogen prior to encapsulation. The resulting solution was filled into soft elastic capsules (1000 mg of solution/SEC) to provide a dosage of 200 mg of ritonavir/SEC or (500 mg of solution/SEC) to provide a dosage of 100 mg of ritonavir/SEC.

### Example 12 (capsule)

| Component | % By Weight |
|---|---|
| ritonavir (free base) | 9 |
| compound **V** (free base) | 27 |
| Ethanol (USP, 200 proof) | 10 |
| polyoxyl 35 castor oil (Cremophor® EL) | 10 |
| Oleic acid, 6321, NF | 43.99 |
| Butylated hydroxy toluene (BHT), NF | 0.01 |

### Example 13 (capsule)

| Component | % By Weight |
|---|---|
| ritonavir (free base) | 7.5 |
| compound **V** (free base) | 30 |
| Ethanol (USP, 200 proof) | 10 |
| polyoxyl 35 castor oil (Cremophor® EL) | 10 |
| Oleic acid, 6321, NF | 42.49 |
| Butylated hydroxy toluene (BHT), NF | 0.01 |

### Example 14 (capsule)

| Component | % By Weight |
|---|---|
| ritonavir (free base) | 17.5 |
| compound **V** (free base) | 17.5 |
| Ethanol (USP, 200 proof) | 10 |
| polyoxyl 35 castor oil (Cremophor® EL) | 10 |
| Oleic acid, 6321, NF | 44.99 |
| Butylated hydroxy toluene (BHT), NF | 0.01 |

### Example 15 (capsule)

| Component | % By Weight |
|---|---|
| ritonavir (free base) | 14 |
| compound **V** (free base) | 28 |
| Ethanol (USP, 200 proof) | 10 |
| polyoxyl 35 castor oil (Cremophor® EL) | 10 |
| Oleic acid, 6321, NF | 37.99 |
| Butylated hydroxy toluene (BHT), NF | 0.01 |

### Example 16 (capsule)

| Component | % By Weight |
|---|---|
| ritonavir (free base) | 9 |
| compound **V** (free base) | 27 |
| Ethanol (USP, 200 proof) | 5 |
| polyoxyl 35 castor oil (Cremophor® EL) | 10 |
| Oleic acid, 6321, NF | 48.99 |
| Butylated hydroxy toluene (BHT), NF | 0.01 |

### Example 17 (capsule)

| Component | % By Weight |
|---|---|
| ritonavir (free base) | 7.5 |
| compound **V** (free base) | 30 |
| Ethanol (USP, 200 proof) | 5 |
| polyoxyl 35 castor oil (Cremophor® EL) | 10 |
| Oleic acid, 6321, NF | 47.49 |
| Butylated hydroxy toluene (BHT), NF | 0.01 |

### Example 18 (SEC)

| Component | % By Weight |
|---|---|
| ritonavir (free base) | 5 |
| compound **V** (free base) | 30 |
| Ethanol (USP, 200 proof) | 10 |
| polyoxyl 35 castor oil (Cremophor® EL) | 10 |
| Oleic acid, 6321, NF | 44.99 |
| Butylated hydroxy toluene (BHT), NF | 0.01 |

The mixing tank was purged with nitrogen. Oleic acid (449.9 g) and ethanol (100g) were mixed in the tank. The butylated hydroxytoluene (0.1 g) was charged into the tank and mixed until the solution was clear. The ritonavir (50 g) was slowly charged into the tank and mixed until the solution was clear. The Compound **V** (300 g) was slowly charged into the tank and mixed until the solution was clear. The polyoxyl 35 castor oil (100 g) was added to the tank and mixed. The resulting solution was stored at 2-8°C before being filled into soft elastic capsules.

### Example 19A (SEC)

| Component | % By Weight |
|---|---|
| ritonavir (free base) | 15 |
| compound **V** (free base) | 15 |
| Ethanol (USP, 200 proof) | 10 |
| polyoxyl 35 castor oil (Cremophor® EL) | 10 |
| Oleic acid, 6321, NF | 49.99 |
| Butylated hydroxy toluene (BHT), NF | 0.01 |

The mixing tank was purged with nitrogen. Oleic acid (499.9 g) and ethanol (100g) were mixed in the tank. The butylated hydroxytoluene (0.1 g) was charged into the tank and mixed until the solution was clear. The ritonavir (150 g) was slowly charged into the tank and mixed until the solution was clear. Compound **V** (150 g) was slowly charged into the tank and mixed until the solution was clear. The polyoxyl 35 castor oil (100 g) was added to the tank and mixed. The resulting solution was filled into soft elastic capsules (1.0 g of solution/SEC) to provide a dosage of 150 mg each of ritonavir and compound **V**/SEC.

### Example 19B (SEC)

| Component | % By Weight |
|---|---|
| ritonavir (free base) | 15 |
| compound **V** (free base) | 15 |
| Ethanol (USP, 200 proof) | 10 |
| polyoxyl 35 castor oil (Cremophor® EL) | 5 |
| Oleic acid, 6321, NF | 54.99 |
| Butylated hydroxy toluene (BHT), NF | 0.01 |

### Example 20 (SEC)

| Component | % By Weight |
|---|---|
| ritonavir (free base) | 15 |
| compound **V** (free base) | 5 |
| Ethanol (USP, 200 proof) | 10 |
| polyoxyl 35 castor oil (Cremophor® EL) | 10 |
| Oleic acid, 6321, NF | 59.99 |
| Butylated hydroxy toluene (BHT), NF | 0.01 |

### Example 21 (SEC)

| Component | % By Weight |
|---|---|
| ritonavir (free base) | 10 |
| compound **V** (free base) | 20 |
| Propylene glycol (USP) | 7.5 |
| Oleic acid, 6321, NF | 52.47 |
| polyoxyl 35 castor oil (Cremophor® EL) | 10 |
| Butylated hydroxy toluene (BHT), NF | 0.03 |

### Example 22 (SEC)

| Component | % By Weight |
|---|---|
| ritonavir (free base) | 10 |
| compound **V** (free base) | 20 |
| Propylene glycol (USP) | 6 |
| Oleic acid, 6321, NF | 53.97 |
| polyoxyl 35 castor oil (Cremophor® EL) | 10 |
| Butylated hydroxy toluene (BHT), NF | 0.03 |

### Example 23 (SEC)

| Component | % By Weight |
|---|---|
| ritonavir (free base) | 11 |
| compound **V** (free base) | 22 |
| Propylene glycol (USP) | 7.5 |
| Oleic acid, 6321, NF | 49.47 |
| po(yoxyl 35 castor oil (Cremophor® EL) | 10 |
| Butylated hydroxy toluene (BHT), NF | 0.03 |

### Example 24 (SEC)

| Component | % By Weight |
|---|---|
| ritonavir (free base) | 9 |
| compound **V** (free base) | 27 |
| Propylene glycol (USP) | 7.5 |
| Oleic acid, 6321, NF | 46.47 |
| polyoxyl 35 castor oil (Cremophor® EL) | 10 |
| Butylated hydroxy toluene (BHT), NF | 0.03 |

### Example 25 (SEC)

| Component | % By Weight |
|---|---|
| ritonavir (free base) | 6.5 |
| compound **V** (free base) | 32.5 |
| Propylene glycol (USP) | 7.5 |
| Oteic acid, 6321, NF | 43.47 |
| polyoxyl 35 castor oil (Cremophor® EL) | 10 |
| Butylated hydroxy toluene (BHT), NF | 0.03 |

### Example 26 (SEC)

| Component | % By Weight |
|---|---|
| ritonavir (free base) | 4.4 |
| compound **V** (free base) | 35 |
| Propylene glycol (USP) | 7.5 |
| Oleic acid, 6321, NF | 53.07 |
| polyoxyl 35 castor oil (Cremophor® EL) | 10 |
| Butylated hydroxy toluene (BHT), NF | 0.03 |

### Example 27 (SEC)

| Component | % By Weight |
|---|---|
| ritonavir (free base) | 5 |
| compound **V** (free base) | 30 |
| Propylene glycol (USP) | 7.5 |
| Oleic acid, 6321, NF | 47.47 |
| polyoxyl 35 castor oil (Cremophor® EL) | 10 |
| Butylated hydroxy toluene (BHT), NF | 0.03 |

### Example 28 (SEC)

| Component | % By Weight |
|---|---|
| ritonavir (free base) | 5 |
| compound **V** (free base) | 30 |
| Propylene glycol (USP) | 6 |
| Oleic acid, 6321, NF | 48.97 |
| polyoxyl 35 castor oil (Cremophor® EL) | 10 |
| Butylated hydroxy toluene (BHT), NF | 0.03 |

### Example 29 (SEC)

| Component | % By Weight |
|---|---|
| ritonavir (free base) | 10 |
| compound **V** (free base) | 20 |
| Propylene glycol (USP) | 7.5 |
| Oleic acid, 6321, NF | 52.47 |
| polyoxyl 35 castor oil (Cremophor® EL) | 10 |
| Butylated hydroxy toluene (BHT), NF | 0.03 |

The mixing tank was purged with nitrogen. Oleic acid (524.7 g) and propylene glycol (75g) were mixed in the tank. The butylated hydroxytoluene (0.3 g) was charged into the tank and mixed until the solution was clear. The ritonavir (100 g) was slowly charged into the tank and mixed until the solution was clear. Heat was applied as necessary. The polyoxyl 35 castor oil (100 g) was added to the tank and mixed. Compound **V** (200 g) was slowly charged into the tank and mixed until the solution was clear. Heat was applied as necessary. The resulting solution was stored at 2-8°C before being filled into soft elastic capsules.

### Example 30 (SEC)

| Component | % By Weight |
|---|---|
| ritonavir (free base) | 5.5 |
| compound **V** (free base) | 33 |
| Propylene glycol (USP) | 7.5 |
| Oleic acid, 6321, NF | 43.97 |
| polyoxyl 35 castor oil (Cremophor® EL) | 10 |
| Butylated hydroxy toluene (BHT), NF | 0.03 |

### Example 31 (SEC)

| Component | % By Weight |
|---|---|
| ritonavir (free base) | 6.0 |
| compound **V** (free base) | 24 |
| Propylene glycol (USP) | 7.5 |
| Oleic acid, 6321, NF | 52.47 |
| polyoxyl 35 castor oil (Cremophor® EL) | 10 |
| Butylated hydroxy toluene (BHT), NF | 0.03 |

The mixing tank was purged with nitrogen. Oleic acid (524.7 g) and butylated hydroxytoluene (0.3 g) were charged into the tank and mixed. Propylene glycol (75.0 g) was charged into the tank. The ritonavir (60 g) was slowly charged into the tank and mixed until the solution was clear. Heat may be applied as necessary. The polyoxyl 35 castor oil (100 g) was added to the tank and mixed. Compound **V** (240 g) was slowly charged into the tank and mixed until the solution was clear. Heat may be applied as necessary. The resulting solution was filled into soft elastic capsules (1.0 g of solution/SEC) to provide a dosage of 60 mg of ritonavir and 240 mg of compound V/SEC.

### Example 32 (SEC)

| Component | % By Weight |
|---|---|
| ritonavir (free base) | 5 |
| compound **V** (free base) | 25 |
| Propylene glycol (USP) | 7.5 |
| Oleic acid, 6321, NF | 52.47 |
| polyoxyl 35 castor oil (Cremophor® EL) | 10 |
| Butylated hydroxy toluene (BHT), NF | 0.03 |

### Example 33 (SEC)

| Component | % By Weight |
|---|---|
| ritonavir (free base) | 8 |
| compound **V** (free base) | 24 |
| Propylene glycol (USP) | 7.5 |
| Oleic acid, 6321, NF | 50.47 |
| polyoxyl 35 castor oil (Cremophor® EL) | 10 |
| Butylated hydroxy toluene (BHT), NF | 0.03 |

### Example 34 (SEC)

| Component | % By Weight |
|---|---|
| ritonavir (free base) | 8.25 |
| compound **V** (free base) | 22 |
| Propylene glycol (USP) | 7.5 |
| Oleic acid, 6321, NF | 52.22 |
| polyoxyl 35 castor oil (Cremophor® EL) | 10 |
| Butylated hydroxy toluene (BHT), NF | 0.03 |

### Example 35 (SEC)

| Component | % By Weight |
|---|---|
| ritonavir (free base) | 20 |
| Propylene glycol (USP) | 5 |
| Ethanol (USP, 200 proof) | 5 |
| Oleic acid, 6321, NF | 69.99 |
| Butylated hydroxy toluene (BHT), NF | 0.01 |

### Example 36 (SEC)

| Component | % By Weight |
|---|---|
| ritonavir (free base) | 10 |
| compound **V** (free base) | 20 |
| Ethanol (USP, 200 proof) | 10 |
| polyoxyl 35 castor oil (Cremophor® EL) | 10 |
| Oleic acid, 6321, NF | 49.99 |
| Butylated hydroxy toluene (BHT), NF | 0.01 |

The mixing tank was purged with nitrogen. Oleic acid (499.9 g) and ethanol (100g) were mixed in the tank. The butylated hydroxytoluene (0.1 g) was charged into the tank and mixed until the solution was clear. The ritonavir (100 g) was slowly charged into the tank and mixed until the solution was clear. The Compound **V** (200 g) was slowly charged into the tank and mixed until the solution was clear. The polyoxyl 35 castor oil (100 g) was added to the tank and mixed. The resulting solution was stored at 2-8°C before being filled into soft elastic capsules.

### Example 37 (SEC)

| Component | % By Weight |
|---|---|
| ritonavir (free base) | 13 |
| compound **V** (free base) | 17 |
| Ethanol (USP, 200 proof) | 10 |
| polyoxyl 35 castor oil (Cremophor® EL) | 10 |
| Oleic acid, 6321, NF | 49.99 |
| Butylated hydroxy toluene (BHT), NF | 0.01 |

### Example 38 (SEC)

| Component | % By Weight |
|---|---|
| ritonavir (free base) | 13 |
| compound **V** (free base) | 17 |
| Propylene glycol (USP) | 7.5 |
| polyoxyl 35 castor oil (Cremophor® EL) | 10 |
| Oleic acid, 6321, NF | 52.47 |
| Butylated hydroxy toluene (BHT), NF | 0.03 |

### Example 39 (SEC)

| Component | % By Weight |
|---|---|
| ritonavir (free base) | 15 |
| compound **V** (free base) | 15 |
| Propylene glycol (USP) | 7.5 |
| polyoxyl 35 castor oil (Cremophor® EL) | 10 |
| Oleic acid, 6321, NF | 52.47 |
| Butylated hydroxy toluene (BHT), NF | 0.03 |

### Example 40

### (2S,3S,5S)-5-(N-(N-((N-Methyl-N-((2-isopropyl-4-thiazolyl)methyl)-amino)carbonyl)valinyl)amino)-2-(N-((5-thiazolyl)methoxycarbonyl)amino)-1,6-diphenyl-3-hydroxyhexane (ritonavir: compound III)

Compound **III** can be prepared according to the procedures disclosed in U.S. Patent No. 5,541,206, issued July 30, 1996, U.S. Patent No. 5,491,253, issued February 13, 1996, and U.S. Patent No. 5,567,823, issued October 22, 1996.

### Example 41

### (2S, 3S, 5S)-2-(2,6-Dimethylphenoxyacetyl) amino-3-hydroxy-5-[2S-(1-tetrahydro-pyrimid-2-onyl)-3-methyl butanoyl] amino-1,6-diphenylhexane (compound V)

Compound **V** can be prepared according to the methods disclosed in International Patent Application No. WO97/21685, published June 19, 1997.

### Protocol For Oral Bioavailability Studies

Dogs (beagle dogs, mixed sexes, weighing 7-14 kg) were fasted overnight prior to dosing, but were permitted water ad libitum. Each dog received a 100 µg/kg subcutaneous dose of histamine approximately 30 minutes prior to dosing. Each dog received a single solid dosage form corresponding to a 5 mg/kg dose of the drug. The dose was followed by approximately 10 milliliters of water. Blood samples were obtained from each animal prior to dosing and 0.25, 0.5, 1.0,1.5, 2, 3, 4, 6, 8, 10 and 12 hours after drug administration. The plasma was separated from the red cells by centrifugation and frozen (-30°C) until analysis. Concentrations of parent drug were determined by reverse phase HPLC with low wavelength UV detection following liquid-liquid extraction of the plasma samples. The parent drug area under the curve was calculated by the trapezoidal method over the time course of the study. The absolute bioavailability of each test composition was calculated by comparing the area under the curve after oral dosing to that obtained from a single intravenous dose. Each capsule or capsule composition was evaluated in a group containing at least six dogs; the values reported are averages for each group of dogs. The average bioavailability data for the compositions of the Examples is shown in Table I.

**TABLE 1**

| Example No. | Mean % Bioavailability |
|---|---|
| Example 1 | 0.0 |
| Example 2 | 0.0 |
| Example 3 | 2.5 |
| Example 4 | 39 |
| Example 5 | 38.8 |
| Example 6 | 39.6 |
| Example 7 | 55.7 |
| Example 8 | 40.3 |
| Example 9 | 61.9 |

This data indicates that solution compositions provided significantly better bioavailability than non-formulated compound **III**. Additionally, the solution composition, encapsulated in hard gelatin capsule or soft elastic capsule, demonstrated greatly improved bioavailability.

Compounds **I, II, III, IV** and **V** are inhibitors of HIV protease. They are useful for inhibiting an HIV infection and treating AIDS in humans. Total daily dose of compound **I, II** or **III** administered to a human in single or divided doses may be in amounts, for example, from 0.001 to 1000 mg/kg body weight daily but more usually 0.1 to 50 mg/kg body weight daily. Total daily dose of compound **IV** or **V** administered to a human in single or divided doses may be in amounts, for example, from 0.001 to 300 mg/kg body weight daily and more usually 0.1 to 20 mg/kg body weight daily. Dosage unit compositions may contain such amounts of submultiples thereof to make up the daily dose. It will be understood, however, that the specific dose level for any particular patient will depend upon a variety of factors including the age, body weight, general health, sex, diet, time of administration, rate of excretion, drugs administered in combination and the severity of the particular disease undergoing therapy.

## Claims

1. A pharmaceutical composition which is a solution comprising
(a) an HIV protease inhibiting compound of the formula 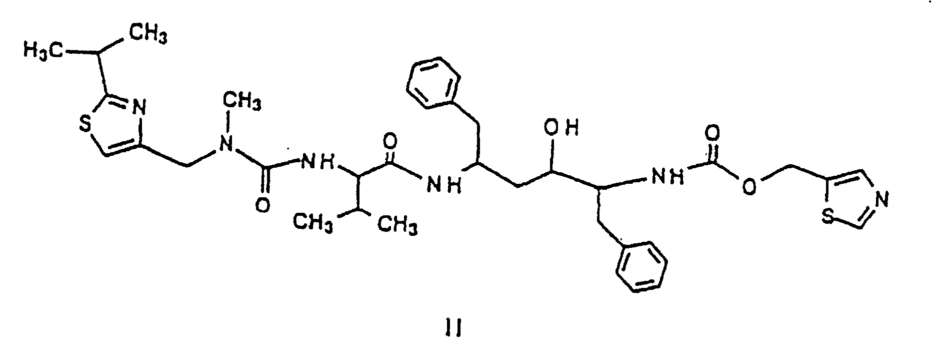 or a pharmaceutically acceptable salt thereof, optionally in combination with another HIV protease inhibiting compound, or a pharmaceutically acceptable salt thereof, in the total amount of from 1% to 50% by weight of the total solution,
(b) a pharmaceutically acceptable organic solvent which comprises (i) a pharmaceutically acceptable fatty acid selected from the group consisting of saturated, mono-unsaturated and di-unsaturated C₁₂ to C₁₈ carboxylic acids which are liquids at room temperature in the amount of from 30% to 99% by weight of the total solution or (ii) a mixture of (1) a pharmaceutically acceptable fatty acid selected from the group consisting of saturated, mono-unsaturated and di-unsaturated C₁₂ to C₁₈ carboxylic acids which are liquids at room temperature in the amount of from 30% to 99% by weight of the total solution and (2) a pharmaceutically acceptable alcohol in the amount of from 0% to 15% by weight of the total solution and
(c) a pharmaceutically acceptable surfactant in the amount of from 0% to 40% by weight of the total solution.

2. The composition of Claim 1 wherein the solution is encapsulated in a hard gelatin capsule or a soft elastic gelatin capsule.

3. The composition of Claim 1 wherein the solvent comprises (1) a pharmaceutically acceptable fatty acid selected from the group consisting of saturated, mono-unsaturated and di-unsaturated C₁₂ to C₁₈ carboxylic acids which are liquids at room temperature in the amount of from 40% to 70% by weight of the total solution and (2) ethanol or propylene glycol in the amount of from 1% to 15% by weight of the tctal solution or a mixture of ethanol and propylene glycol in the amount of from 1% to 15% by weight of the total solution.

4. The composition of Claim 1 wherein the solvent comprises (1) a pharmaceutically acceptable fatty acid selected from the group consisting of saturated, mono-unsaturated and di-unsaturated C₁₂ to C₁₈ carboxylic acids which are liquids at room temperature in the amount of from 40% to 70% by weight of the total solution and (2) ethanol in the amount or from 10% to 12% by weight of the total solution or propylene glycol in the amount of from 5% to 10% by weight of the total solution or a mixture of ethanol and propylene glycol in the amount of from 5% to 15% by weight of the total solution.

5. The composition of Claim 1 wherein the solvent comprises (1) oleic acid in the amount of from 40% to 70% by weight of the total solution and (2) ethanol in the amount of from 10% to 12% by weight of the total solution or propylene glycol in the amount of from 5% to 10% by weight of the total solution or a mixture of ethanol and propylene glycol in the amount of from 10% to 15% by weight of the total solution.

6. The composition of Claim 1 wherein the HIV protease inhibiting compound is (2S,3S,5S)-5-(N-(N-((N-methyl-N-((2-isopropyl-4-thiazolyl)methyl)-amino)carbonyl)valinyl)amino)-2-(N-((5-thiazolyl)methoxycarbonyl)amino)-1,6-diphenyl-3-hydroxyhexane (ritonavir). or a pharmaceutically acceptable salt thereof.

7. The composition of Claim 1 wherein the HIV protease inhibiting compound in combination with the compound of formula II is selected from the group consisting of
N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)-piperazinyl))-pentaneamide (indinavir);
N-tert-butyl-decahydro-2-(2(R)-hydroxy-4-phenyl-3(S)-))N-(2-quinolylcarbonyl)-L-asparaginyl)amino)butyl)-(4aS,8aS)-isoquinoline-3(S)-carboxamide (saquinavir); 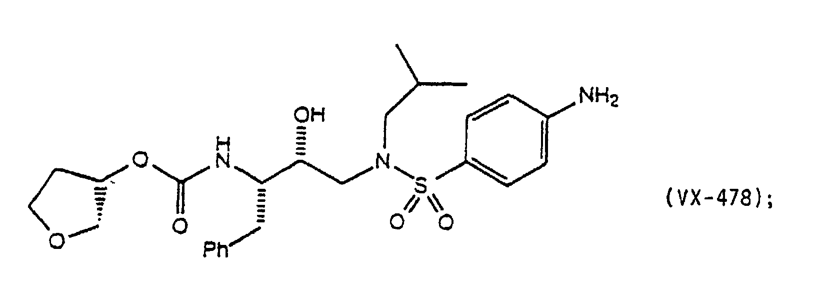 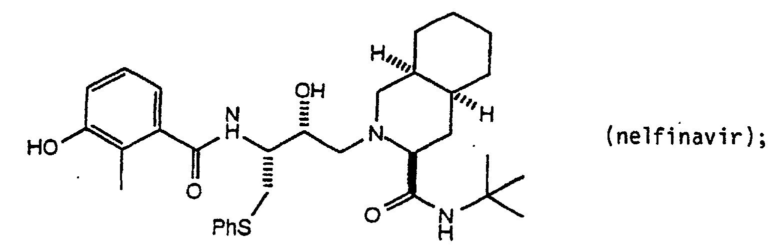 and a compound of the formula 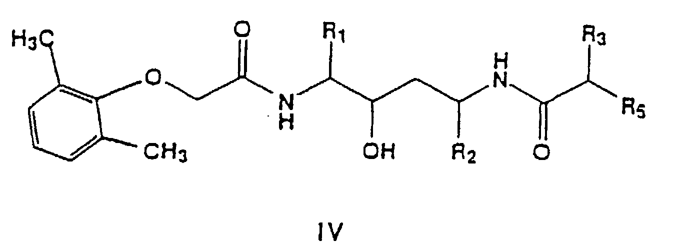 wherein R₁ is benzyl, R₂ is benzyl or C₁-C₆ alkyl, R₃ is C₁-C₆ alkyl and R₅ is 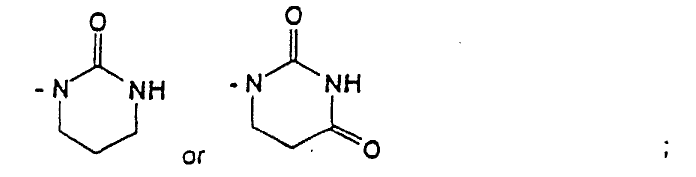 or a pharmaceutically acceptable salt of any of the above.

8. The composition of Claim 1 wherein the HIV protease inhibiting compound is ritonavir or a combination of ritonavir and another HIV protease inhibiting compound.

9. The composition of Claim 1 wherein the combination of HIV protease inhibiting compounds is:
ritonavir and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane;
ritonavir and indinavir;
ritonavir and saquinavir;
ritonavir and nelfinavir; or
ritonavir and VX-478.

10. The composition of Claim 1 wherein the HIV protease inhibiting compound is ritonavir or a combination of ritonavir and (2S,3S,5S)-2-(2,6-dimethylphenoxyacetyl)amino-3-hydroxy-5-(2S-(1-tetrahydro-pyrimid-2-onyl)-3-methyl butanoyl) amino-1,6-diphenylhexane.

11. The composition of Claim 1 comprising
(a) ritonavir in the amount of from 1% to 30% by weight of the total solution,
(b) a pharmaceutically acceptable organic solvent which comprises (i) a pharmaceutically acceptable fatty acid selected from the group consisting of saturated, mono-unsaturated and di-unsaturated C₁₂ to C₁₈ carboxylic acids which are liquids at room temperature in the amount of from 30% to 99% by weight of the total solution or (ii) a mixture of (1) a pharmaceutically acceptable fatty acid selected from the group consisting of saturated, mono-unsaturated and di-unsaturated C₁₂ to C₁₈ carboxylic acids which are liquids at room temperature in the amount of from 30% to 99% by weight of the total solution and (2) a pharmaceutically acceptable alcohol in the amount of from 0% to 15% by weight of. the total solution and
(c) a pharmaceutically acceptable surfactant in the amount of from 0% to 20% by weight of the total solution.

12. The composition of Claim 11 wherein the solution is encapsulated in a soft elastic gelatin capsule (SEC).

13. The composition of Claim 11 comprising
(a) ritonavir in the amount of from 5% to 25% by weight of the total solution,
(b) a pharmaceutically acceptable organic solvent which comprises (i) a pharmaceutically acceptable fatty acid selected from the group consisting of saturated, mono-unsaturated and di-unsaturated C₁₂ to C₁₈ carboxylic acids which are liquids at room temperature in the amount of from 30% to 70% by weight of the total solution or (ii) a mixture of (1) a pharmaceutically acceptable fatty acid selected from the group consisting of saturated, mono-unsaturated and di-unsaturated C₁₂ to C₁₈ carboxylic acids which are liquids at room temperature in the amount of from 30% to 70% by weight of the total solution and (2) a pharmaceutically acceptable alcohol in the amount of from 6% to 12% by weight of the total solution and
(c) a pharmaceutically acceptable surfactant in the amount of from 5% to 10% by weight of the total solution.

14. The composition of Claim 13 wherein the solution is encapsulated in a soft elastic gelatin capsule (SEC).

15. The composition of Claim 1 comprising
(a) ritonavir in the amount of from 1% to 30% by weight of the total solution,
(b) a pharmaceutically acceptable organic solvent which comprises (i) oleic acid in the amount of from 30% to 99% by weight of the total solution or (ii) a mixture of (1) oleic acid in the amount of from 30% to 99% by weight of the total solution and (2) ethanol in the amount of from 0% to 12% by weight of the total solution or propylene glycol in the amount of from 0% to 10% by weight of the total solution or a mixture thereof in the amount of from 0% to 15% by weight of the total solution and
(c) polyoxyl 35 castor oil in the amount of from 0% to 20% by weight of the total solution.

16. The composition of Claim 15 comprising
(a) ritonavir in the amount of from 5% to 25% by weight of the total solution,
(b) a pharmaceutically acceptable organic solvent which comprises (i) oleic acid in the amount of from 30% to 70% by weight of the total solution or (ii) a mixture of (1) oleic acid in the amount of from 30% to 70% by weight of the total solution and (2) ethanol in the amount of from 10% to 12% by weight of the total solution or propylene glycol in the amount of from 5% to 10% by weight of the total solution or a mixture thereof in the amount of from 10% to 15% by weight of the total solution and
(c) polyoxyl 35 castor oil in the amount of from 5% to 10% by weight of the total solution.

17. The composition of Claim 15 wherein the solution is encapsulated in a soft elastic gelatin capsule (SEC).

18. The composition of Claim 15 comprising
(a) ritonavir in the amount of about 20% by weight of the total solution,
(b) a pharmaceutically acceptable organic solvent which comprises a mixture of (1) oleic acid in the amount of from 62% to 64% by weight of the total solution and (2) ethanol in the amount of from 10% to about 12% by weight of the total solution and
(c) polyoxyl 35 castor oil in the amount of about 6% by weight of the total solution.

19. The composition of Claim 15 comprising
(a) ritonavir in the amount of about 20% by weight of the total solution,
(b) a pharmaceutically acceptable organic solvent which comprises a mixture of (1) oleic acid in the amount of about 65% by weight of the total solution and (2) ethanol in the amount of about 10% by weight of the total solution and
(c) polyoxyl 35 castor oil in the amount of about 5% by weight of the total solution.

20. The composition of Claim 15 comprising
(a) ritonavir in the amount of about 20% by weight of the total solution,
(b) a pharmaceutically acceptable organic solvent which comprises a mixture of (1) oleic acid in the amount of about 60% by weight of the total solution and (2) ethanol in the amount of about 10% by weight of the total solution and
(c) polyoxyl 35 castor oil in the amount of about 10% by weight of the total solution.

21. The composition of Claim 1 comprising
(a) a mixture of ritonavir in the amount of from 1% to 30% by weight of the total solution and another HIV protease inhibitor in the amount of from 1% to 50% by weight of the total solution,
(b) a pharmaceutically acceptable organic solvent which comprises (i) a pharmaceutically acceptable fatty acid selected from the group consisting of saturated, mono-unsaturated and di-unsaturated C₁₂ to C₁₈ carboxylic acids which are liquids at room temperature in the amount of from 30% to 98% by weight of the total solution or (ii) a mixture of (1) a pharmaceutically acceptable fatty acid selected from the group consisting of saturated, mono-unsaturated and di-unsaturated C₁₂ to C₁₈ carboxylic acids which are liquids at room temperature in the amount of from 30 % to 98% by weight of the total solution and (2) a pharmaceutically acceptable alcohol in the amount of from 0% to 15% by weight of the total solution and
(c) a pharmaceutically acceptable surfactant in the amount of from 0% to 20% by weight of the total solution.

22. The composition of Claim 21 comprising
(a) a mixture of ritonavir in the amount of from . 5% to 25% by weight of the total solution and another HIV protease inhibitor in the amount of from 5% to 40% by weight of the total solution,
(b) a pharmaceutically acceptable organic solvent which comprises (i) a pharmaceutically acceptable fatty acid selected from the group consisting of saturated, mono-unsaturated and di-unsaturated C₁₂ to C₁₈ carboxylic acids which are liquids at room temperature in the amount of from 30% to 70% by weight of the total solution or (ii) a mixture of (1) a pharmaceutically acceptable fatty acid selected from the group consisting of saturated, mono-unsaturated and di-unsaturated C₁₂ to C₁₈ carboxylic acids which are liquids at room temperature in the amount of from 30% to 70% by weight of the total solution and (2) a pharmaceutically acceptable alcohol in the amount of from 6% to 12% by weight of the total solution and
(c) a pharmaceutically acceptable surfactant in the amount of from 5% to 10% by weight of the total solution.

23. The composition of Claim 21 comprising
(a) a mixture of ritonavir in the amount of from 1% to 30% by weight of the total solution and another HIV protease inhibitor in the amount of from 1% to 50% by weight of the total solution,
(b) a pharmaceutically acceptable organic solvent which comprises (i) oleic acid in the amount of from 30% to 98% by weight of the total solution or (ii) a mixture of (1) oleic acid in the amount of from 30% to 98% by weight of the total solution and (2) ethanol in the amount of from about 0% to 12% by weight of the total solution or propylene glycol in the amount of from 0% to 10% by weight of the total solution or a mixture thereof in the amount of from 0% to 15% by weight of the total solution and
(c) polyoxyl 35 castor oil in the amount of from 0% to 20% by weight of the total solution.

24. The composition of Claim 23 comprising
(a) a mixture of ritonavir in the amount of from 5% to 25% by weight of the total solution and another HIV protease inhibitor in the amount of from 5% to 40% by weight of the total solution,
(b) a pharmaceutically acceptable organic solvent which comprises (i) oleic acid in the amount of from 30% to 70% by weight of the total solution or (ii) a mixture of (1) oleic acid in the amount of from 30% to 70% by weight of the total solution and (2) ethanol in the amount of from 10% to 12% by weight of the tctal solution or propylene glycol in the amount of from 5% to 10% by weight of the total solution or a mixture thereof in the amount of from 10% to 15% by weight of the total solution and
(c) polyoxyl 35 castor oil in the amount of from 5% to 10% by weight of the total solution.

25. The composition of Claim 23 comprising
(a) a mixture of ritonavir in the amount of from 1% to 30% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of from 1 % to 50% by weight of the total solution,
(b) a pharmaceutically acceptable organic solvent which comprises a mixture of (1) oleic acid in the amount of from 30% to 88% by weight of the total solution and (2) ethanol in the amount of about 10% by weight of the total solution and
(c) polyoxyl 35 castor oil in the amount of about 10% by weight of the total solution.

26. The composition of Claim 25 comprising
(a) a mixture of ritonavir in the amount of from 5% to 25% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of from 5% to 40% by weight of the total solution,
(b) a pharmaceutically acceptable organic solvent which comprises a mixture of (1) oleic acid in the amount of from 40% to 65% by weight of the total solution and (2) ethanol in the amount of about 10% by weight of the total solution and
(c) polyoxyl 35 castor oil in the amount of about 10% by weight of the total solution, in .

27. The composition of Claim 21 comprising
(a) a mixture of ritonavir in the amount of about 5% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the. amount of about 30% by weight of the total solution,
(b) a pharmaceutically acceptable organic solvent which comprises a mixture of (1) oleic acid in the amount of about 45% by weight of the total solution and (2) ethanol in the amount of about 10% by weight of the total solution and
(c) polyoxyl 35 castor oil in the amount of about 10% by weight of the total solution.

28. The composition of Claim 21 comprising
(a) a mixture of ritonavir in the amount of about 15% by weight of the total solution and (2S, 3S, 5S)-2-(2,6-dimethylphenoxyacetyl) amino-3-hydroxy-5-(2S-(1-tetrahydro-pyrimld-2-onyl)-3-methyl butanoyl) amino-1,6-diphenylhexane in the amount of about 15% by weight of the total solution,
(b) a pharmaceutically acceptable organic solvent which comprises a mixture of (1) oleic acid in the amount of about 50% by weight of the total solution and (2) ethanol in the amount of about 10% by weight of the total solution and
(c) polyoxyl 35 castor oil in the amount of about 10% by weight of the total solution.

29. The composition of Claim 21 comprising
(a) a mixture of ritonavir in the amount of about 15% by weight of the total solution and (2S, 3S, 5S)-2-(2,6-dimethylphenoxyacetyl) amino-3-hydroxy-5-(2S-(1-tetrahydro-pyrimid-2-onyl)-3-methylbutanoyl) amino-1,6-diphenylhexane in the amount of about 5% by weight of the total solution,
(b) a pharmaceutically acceptable organic solvent which comprises a mixture of (1) oleic acid in the amount of about 60% by weight of the total solution and (2) ethanol in the amount of about 10% by weight of the total solution and
(c) polyoxyl 35 castor oil in the amount of about 10% by weight of the total solution.

30. The composition of Claim 21 comprising a mixture of ritonavir and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane, the ratio (w/w) of ritonavir to (2S, 3S, 5S)-2-(2,6-dimethylphenoxyacetyl) amino-3-hydroxy-5-(2S-(7-tetrahydro-pyrimid-2-ony()-3-methyl butanoyl) amino-1,6-diphenylhexane being from 1:16 to 5:1.

31. The composition of Claim 30 comprising a mixture of ritonavir and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane, the ratio (w/w) of ritonavir to (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane being from 1:8 to 3:1.

32. The composition of Claim 1 comprising
(a) a mixture of ritonavir in the amount of from 1% to 30% by weight of the total solution and another HIV protease inhibiting compound in the amount of from 1% to 50% by weight of the total solution,
(b) a pharmaceutically' acceptable organic solvent which comprises a mixture of (1) oleic acid in the amount of from 30% to 88% by weight of the total solution and (2) propylene glycol in the amount of from 5% to 10% by weight of the total solution and
(c) polyoxyl 35 castor oil in the amount of about 10% by weight of the total solution.

33. The composition of Claim 32 comprising
(a) a mixture of ritonavir in the amount of from 5% to 25% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of from 5% to 40% by weight of the total solution,
(b) a pharmaceutically acceptable organic solvent which comprises a mixture of (1) oleic acid in the amount of from 40% to 65% by weight of the total solution and (2) propylene glycol in the amount of from 6% to 8% by weight of the total solution and
(c) polyoxyl 35 castor oil in the amount of about 10% by weight of the total solution.

34. The composition of Claim 32 comprising
(a) a mixture of ritonavir in the amount of from 1% to 30% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of from 1% to 50% by weight of the total solution,
(b) a pharmaceutically acceptable organic solvent which comprises a mixture of (1) oleic acid in the amount of from 30% to 88% by weight of the total solution and (2) propylene glycol in the amount of from 5% to 10% by weight of the total solution and
(c) polyoxyl 35 castor oil in the amount of about 10% by weight of the total solution.

35. The composition of Claim 34 comprising
(a) a mixture of ritonavir in the amount of from 5% to 25% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of from 5% to 40% by weight of the total solution,
(b) a pharmaceutically acceptable organic solvent which comprises a mixture of (1) oleic acid in the amount of from 40% to 65% by weight cf the total solution and (2) propylene glycol in the amount of from 6% to 8% by weight of the total solution and
(c) polyoxyl 35 castor oil in the amount of about 10% by weight of the total solution.

36. The composition of Claim 35 comprising
(a) a mixture of ritonavir in the amount of about 6.0% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 24% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil'in the amount of about 10% by weight of the total solution.

37. The composition of Claim 35 comprising a solution of (a) a mixture of ritonavir in the amount of about 5% by weight of the total solution and (2S, 3S, 5S)-2-(2,6-dimethylphenoxyacetyl) amino-3-hydroxy-5-(2S-(1-tetrahydropyrimid-2-onyl)-3-methyl butanoyl) amino-1,6-diphenylhexane in the amount of about 25% by weight of the total solution and (b) polyoxyl 35 castor oil in the amount of about 10% by weight of the total solution, in a pharmaceutically acceptable organic solvent which comprises a mixture of (1) oleic acid in the amount of about 52.5% by weight of the total solution and (2) propylene glycol in the amount of about 7.5% by weight of the total solution.

38. The composition of Claim 35 comprising
(a) a mixture of ritonavir in the amount of about 8% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 24% by weight of the total solution,
(b) a pharmaceutically acceptable organic solvent which comprises a mixture of (1) oleic acid in the amount of about 50.5% by weight of the total solution and (2) propylene glycol in the amount of about 7.5% by weight of the total solution and
(c) polyoxyl 35 castor oil in the amount of about 10% by weight of the total solution.

39. The composition of Claim 35 comprising
(a) a mixture of ritonavir in the amount of about 8.25% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 22% by weight of the total solution,
(b) a pharmaceutically acceptable organic solvent which comprises a mixture of (1) oleic acid in the amount of about 52.25% by weight of the total solution and (2) propylene glycol in the amount of about 7.5% by weight of the total solution and
(c) polyoxyl 35 castor oil in the amount of about 10% by weight of the total solution.

40. The composition of Claim 35 comprising
(a) a mixture of ritonavir in the amount of about 5% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 30% by weight of the total solution,
(b) a pharmaceutically acceptable organic solvent which comprises a mixture of (1) oleic acid in the amount of about 47.5% by weight of the total solution and (2) propylene glycol in the amount of about 7.5% by weight of the total solution and
(c) polyoxyl 35 castor oil in the amount of about 10% by weight of the total solution.

41. The composition of Claim 35 comprising
(a) a mixture of ritonavir in the amount of about 15% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 15% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution.

42. The composition of Claim 35 comprising
(a) a mixture of ritonavir in the amount of about 13% by weight of the total solution and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane in the amount of about 17% by weight of the total solution,
(b) a pharmaceutically acceptable organic solvent which comprises a mixture of (1) oleic acid in the amount of about 52.5% by weight of the total solution and (2) propylene glycol in the amount of about 7.5% by weight of the total solution and
(c) polyoxyl 35 castor oil in the amount of about 10% by weight of the total solution.

43. The composition of Claim 35 comprising a mixture of ritonavir and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane, the ratio (w/w) of ritonavir to (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane being from 1:16 to 5:1.

44. The composition of Claim 41 comprising a mixture of ritonavir and (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane, the ratio (w/w) of ritonavir to (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexane being from 1:8 to 3:1.

## Patentansprüche

1. Eine pharmazeutische Zusammensetzung, welche eine Lösung ist, die folgendes umfaßt:
(a) eine HIV-Protease-hemmende Verbindung mit der Formel 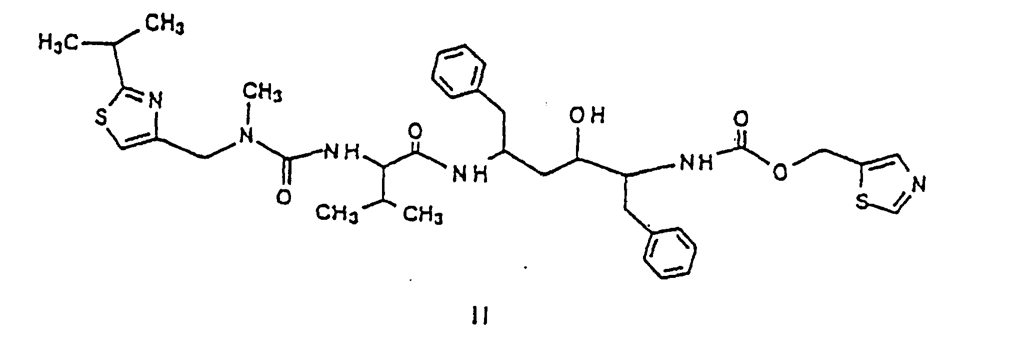 oder ein pharmazeutisch verträgliches Salz davon, wahlweise in Kombination mit einer anderen HIV-Protease-hemmenden Verbindung oder einem pharmazeutisch verträglichen Salz davon, in der Gesamtmenge von 1 Gew% bis 50 Gew% der gesamten Lösung,
(b) ein pharmazeutisch verträgliches organisches Lösungsmittel, welches (i) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 99 Gew% der gesamten Lösung, oder (ii) eine Mischung aus (1) einer pharmazeutisch verträglichen Fettsäure gewählt aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 99 Gew% der gesamten Lösung, und (2) einem pharmazeutisch verträglichen Alkohol in der Menge von 0 Gew% bis 15 Gew% der gesamten Lösung, und
(c) einen pharmazeutisch verträglichen oberflächenaktiven Stoff in der Menge von 0 Gew% bis 40 Gew% der gesamten Lösung.

2. Die Zusammensetzung von Anspruch 1, worin die Lösung in eine Hartgelatinekapsel oder eine weiche elastische Gelatinekapsel eingekapselt ist.

3. Die Zusammensetzung von Anspruch 1, worin das Lösungsmittel (1) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 40 Gew% bis 70 Gew% der gesamten Lösung, und (2) Ethanol oder Propylenglykol in der Menge von 1 Gew% bis 15 Gew% der gesamten Lösung oder eine Mischung aus Ethanol und Propylenglykol in der Menge von 1 Gew% bis 15 Gew% der gesamten Lösung.

4. Die Zusammensetzung von Anspruch 1, worin das Lösungsmittel (1) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 40 Gew% bis 70 Gew% der gesamten Lösung, und (2) Ethanol in der Menge von 10 Gew% bis 12 Gew% der gesamten Lösung, oder Propylenglykol in der Menge von 5 Gew% bis 10 Gew% der gesamten Lösung, oder eine Mischung aus Ethanol und Propylenglykol in der Menge von 5 Gew% bis 15 Gew% der gesamten Lösung.

5. Die Zusammensetzung von Anspruch 1, worin das Lösungsmittel (1) Ölsäure in der Menge von 40 Gew% bis 70 Gew% der gesamten Lösung, und (2) Ethanol in der Menge von 10 Gew% bis 12 Gew% der gesamten Lösung, oder Propylenglykol in der Menge von 5 Gew% bis 10 Gew% der gesamten Lösung, oder eine Mischung aus Ethanol und Propylenglykol in der Menge von 10 Gew% bis 15 Gew% der gesamten Lösung umfaßt.

6. Die Zusammensetzung von Anspruch 1, worin die HIV-Protease-hemmende Verbindung (2S,3S,5S)-5-(N-(N-((N-methyl-N-((2-isopropyl-4-thiazolyl)methyl)-amino)carbonyl)valinyl)amino)-2-(N-((5-thiazolyl)methoxycarbonyl)amino)-1,6-diphenyl-3-hydroxyhexan (Ritonavir) oder ein pharmazeutisch verträgliches Salz davon ist.

7. Die Zusammensetzung von Anspruch 1, worin die HIV-Protease-hemmende Verbindung in Kombination mit der Verbindung von Formel II gewählt ist aus der Gruppe bestehend aus
N-(2(R)-Hydroxy-1(S)-indanyl)-2(R)-phenylmethyl-4(S)-hydroxy-5-(1-(4-(3-pyridylmethyl)-2(S)-N'-(t-butylcarboxamido)-piperazinyl))-pentaneamid(indinavir);
N-tert-Butyl-decahydro-2-(2 (R)-hydroxy-4-phenyl-3 (S)-))N-(2-chinolylcarbonyl)-L-asparaginyl)amino)butyl)-(4aS,8aS)-isochinolin-3(S)-carboxamid(saquinavir); 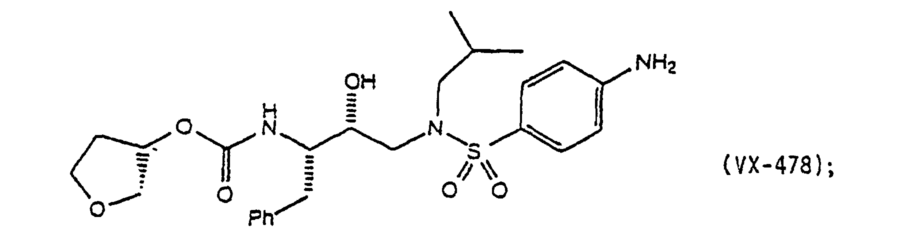 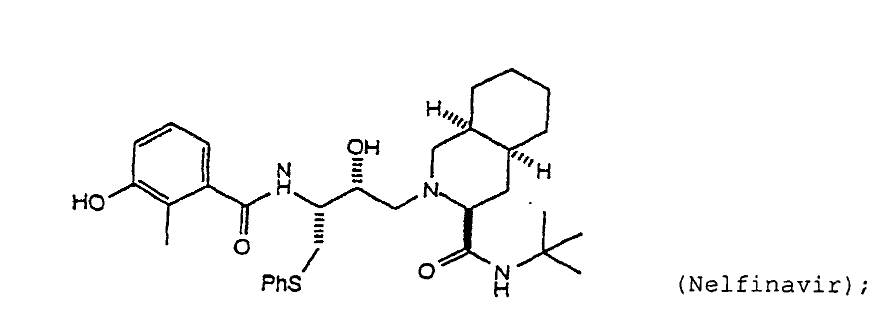 und eine Verbindung mit der Formel 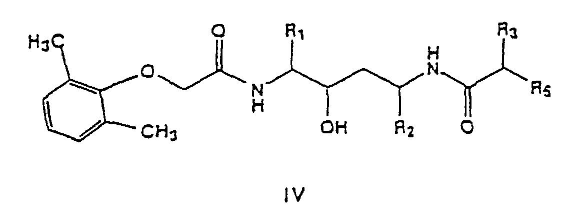 worin R₁ Benzyl ist, R₂ ist Benzyl oder C₁-C₆ Alkyl, R₃ ist C₁-C₆ Alkyl und R₅ ist 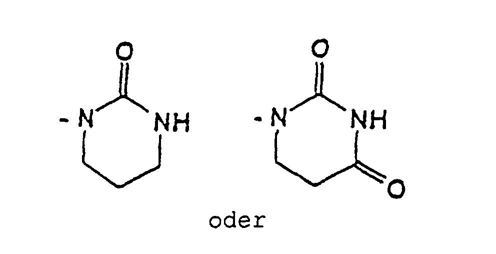 oder ein pharmazeutisch verträgliches Salz von irgendeinem der obigen.

8. Die Zusammensetzung von Anspruch 1, worin die HIV-Protease-hemmende Verbindung Ritonavir ist, oder eine Kombination aus Ritonavir und einer anderen HIV-Protease-hemmenden Verbindung.

9. Die Zusammensetzung von Anspruch 1, worin die Kombination aus HIV-Protease-hemmenden Verbindungen folgende ist:
Ritonavir und (23,3S,53)-2-(2,6-dimethylphenoxyacetyl)amino-3-hydroxy-5-(2S-(1-tetrahydro-pyrimid-2-onyl)-3-methyl butanoyl) amino-1,6-diphenylhexan;
Ritonavir und Indinavir;
Ritonavir und Saquinavir;
Ritonavir und Nelfinavir; oder
Ritonavir und VX-478.

10. Die Zusammensetzung von Anspruch 1, worin die HIV-Protease-hemmende Verbindung Ritonavir oder eine Kombination aus Ritonavir und (2S,3S,5S)-2-(2,6-dimethylphenoxyacetyl)amino-3-hydroxy-5-(23-(1-tetrahydro-pyrimid-2-onyl)-3-methyl butanoyl) amino-1,6-diphenylhexan ist.

11. Die Zusammensetzung von Anspruch 1, die folgendes umfaßt:
(a) Ritonavir in der Menge von 1 Gew% bis 30 Gew% der gesamten Lösung,
(b) ein pharmazeutisch verträgliches organisches Lösungsmittel, welches (i) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 99 Gew% der gesamten Lösung, oder (ii) eine Mischung aus (1) einer pharmazeutisch verträglichen Fettsäure gewählt aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 99 Gew% der gesamten Lösung, und (2) einem pharmazeutisch verträglichen Alkohol in der Menge von 0 Gew% bis 15 Gew% der gesamten Lösung, und
(c) einen pharmazeutisch verträglichen oberflächenaktiven Stoff in der Menge von 0 Gew% bis 20 Gew% der gesamten Lösung.

12. Die Zusammensetzung von Anspruch 11, worin die Lösung in eine weiche elastische Gelatinekapsel (SEC) eingekapselt ist.

13. Die Zusammensetzung von Anspruch 11, die folgendes umfaßt.
(a) Ritonavir in der Menge von 5 Gew% bis 25 Gew% der gesamten Lösung,
(b) ein pharmazeutisch verträgliches organisches Lösungsmittel, welches (i) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 70 Gew% der gesamten Lösung, oder (ii) eine Mischung aus (1) einer pharmazeutisch verträglichen Fettsäure gewählt aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 70 Gew% der gesamten Lösung, und (2) einem pharmazeutisch verträglichen Alkohol in der Menge von 6 Gew% bis 12 Gew% der gesamten Lösung, und
(c) einen pharmazeutisch verträglichen oberflächenaktiven Stoff in der Menge von 5 Gew% bis 10 Gew% der gesamten Lösung.

14. Die Zusammensetzung von Anspruch 13, worin die Lösung in eine weiche elastische Gelatinekapsel (SEC) eingekapselt ist.

15. Die Zusammensetzung von Anspruch 1, die folgendes umfaßt:
(a) Ritonavir in der Menge von 1 Gew% bis 30 Gew% der gesamten Lösung,
(b) ein pharmazeutisch verträgliches organisches Lösungsmittel, welches (i) Ölsäure in der Menge von 30 Gew% bis 99 Gew% der gesamten Lösung, oder (ii) eine Mischung aus (1) Ölsäure in der Menge von 30 Gew% bis 99 Gew% der gesamten Lösung, und (2) Ethanol in der Menge von 0 Gew% bis 12 Gew% der gesamten Lösung, oder Propylenglykol in der Menge von 0 Gew% bis 10 Gew% der gesamten Lösung, oder einer Mischung daraus in der Menge von 0 Gew% bis 15 Gew% der gesamten Lösung, umfaßt und
(c) Polyoxyl 35 Rizinusöl in der Menge von 0 Gew% bis 20 Gew% der gesamten Lösung.

16. Die Zusammensetzung von Anspruch 15, die folgendes umfaßt:
(a) Ritonavir in der Menge von 5 Gew% bis 25 Gew% der gesamten Lösung,
(b) ein pharmazeutisch verträgliches organisches Lösungsmittel, welches (i) Ölsäure in der Menge von 30 Gew% bis 70 Gew% der gesamten Lösung, oder (ii) eine Mischung aus (1) Ölsäure in der Menge von 30 Gew% bis 70 Gew% der gesamten Lösung, und (2) Ethanol in der Menge von 10 Gew% bis 12 Gew% der gesamten Lösung, oder Propylenglykol in der Menge von 5 Gew% bis 10 Gew% der gesamten Lösung, oder einer Mischung daraus in der Menge von 10 Gew% bis 15 Gew% der gesamten Lösung, umfaßt und
(c) Polyoxyl 35 Rizinusöl in der Menge von 5 Gew% bis 10 Gew% der gesamten Lösung.

17. Die Zusammensetzung von Anspruch 15, worin die Lösung in eine weiche elastische Gelatinekapsel (SEC) eingekapselt ist.

18. Die Zusammensetzung von Anspruch 15, die folgendes umfaßt:
(a) Ritonavir in der Menge von ungefähr 20 Gew% der gesamten Lösung,
(b) ein pharmazeutisch verträgliches organisches Lösungsmittel, welches eine Mischung aus (1) Ölsäure in der Menge von 62 Gew% bis 64 Gew% der gesamten Lösung und (2) Ethanol in der Menge von 10 Gew% bis 12 Gew% der gesamten Lösung umfaßt, und
(c) Polyoxyl 35 Rizinusöl in der Menge von ungefähr 6 Gew% der gesamten Lösung.

19. Die Zusammensetzung von Anspruch 15, die folgendes umfaßt:
(a) Ritonavir in der Menge von ungefähr 20 Gew% der gesamten Lösung,
(b) ein pharmazeutisch verträgliches organisches Lösungsmittel, welches eine Mischung aus (1) Ölsäure in der Menge von ungefähr 65 Gew% der gesamten Lösung und (2) Ethanol in der Menge von ungefähr 10 Gew% der gesamten Lösung umfaßt, und
(c) Polyoxyl 35 Rizinusöl in der Menge von ungefähr 5 Gew% der gesamten Lösung.

20. Die Zusammensetzung von Anspruch 15, die folgendes umfaßt:
(a) Ritonavir in der Menge von ungefähr 20 Gew% der gesamten Lösung,
(b) ein pharmazeutisch verträgliches organisches Lösungsmittel, welches eine Mischung aus (1) Ölsäure in der Menge von ungefähr 60 Gew% der gesamten Lösung und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

21. Die Zusammensetzung von Anspruch 1, die folgendes umfaßt:
(a) eine Mischung aus Ritonavir in der Menge von 1 Gew% bis 30 Gew% der gesamten Lösung und einem anderen HIV Proteaseinhibitor in der Menge von 1 Gew% bis 50 Gew% der gesamten Lösung,
(b) ein pharmazeutisch verträgliches organisches Lösungsmittel, welches (i) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 98 Gew% der gesamten Lösung, oder (ii) eine Mischung aus (1) einer pharmazeutisch verträglichen Fettsäure gewählt aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 98 Gew% der gesamten Lösung, und (2) einem pharmazeutisch verträglichen Alkohol in der Menge von 0 Gew% bis 15 Gew% der gesamten Lösung, und
(c) einen pharmazeutisch verträglichen oberflächenaktiven Stoff in der Menge von 0 Gew% bis 20 Gew% der gesamten Lösung.

22. Die Zusammensetzung von Anspruch 21, die folgendes umfaßt:
(a) eine Mischung aus Ritonavir in der Menge von 5 Gew% bis 25 Gew% der gesamten Lösung und einem anderen HIV Proteaseinhibitor in der Menge von 5 Gew% bis 40 Gew% der gesamten Lösung,
(b) ein pharmazeutisch verträgliches organisches Lösungsmittel, welches (i) eine pharmazeutisch verträgliche Fettsäure umfaßt, die gewählt ist aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 70 Gew% der gesamten Lösung, oder (ii) eine Mischung aus (1) einer pharmazeutisch verträglichen Fettsäure gewählt aus der Gruppe bestehend aus gesättigten, mono-ungesättigten und di-ungesättigten C₁₂ bis C₁₈ Carbonsäuren, welche bei Raumtemperatur Flüssigkeiten sind, in der Menge von 30 Gew% bis 70 Gew% der gesamten Lösung, und (2) einem pharmazeutisch verträglichen Alkohol in der Menge von 6 Gew% bis 12 Gew% der gesamten Lösung, und
(c) einen pharmazeutisch verträglichen oberflächenaktiven Stoff in der Menge von 5 Gew% bis 10 Gew% der gesamten Lösung.

23. Die Zusammensetzung von Anspruch 21, die folgendes umfaßt:
(a) eine Mischung aus Ritonavir in der Menge von 1 Gew% bis 30 Gew% der gesamten Lösung und einen anderen HIV Proteaseinhibitor in der Menge von 1 Gew% bis 50 Gew% der gesamten Lösung,
(b) ein pharmazeutisch verträgliches organisches Lösungsmittel, welches (i) Ölsäure in der Menge von 30 Gew% bis 98 Gew% der gesamten Lösung, oder (ii) eine Mischung aus (1) Ölsäure in der Menge von 30 Gew% bis 98 Gew% der gesamten Lösung, und (2) Ethanol in der Menge von 0 Gew% bis 12 Gew% der gesamten Lösung, oder Propylenglykol in der Menge von 0 Gew% bis 10 Gew% der gesamten Lösung, oder einer Mischung daraus in der Menge von 0 Gew% bis 15 Gew% der gesamten Lösung, umfaßt und
(c) Polyoxyl 35 Rizinusöl in der Menge von 0 Gew% bis 20 Gew% der gesamten Lösung.

24. Die Zusammensetzung von Anspruch 23, die folgendes umfaßt:
(a) eine Mischung aus Ritonavir in der Menge von 5 Gew% bis 25 Gew% der gesamten Lösung und einen anderen HIV Proteaseinhibitor in der Menge von 5 Gew% bis 40 Gew% der gesamten Lösung,
(b) ein pharmazeutisch verträgliches organisches Lösungsmittel, welches (i) Ölsäure in der Menge von 30 Gew% bis 70 Gew% der gesamten Lösung, oder (ii) eine Mischung aus (1) Ölsäure in der Menge von 30 Gew% bis 70 Gew% der gesamten Lösung, und (2) Ethanol in der Menge von 10 Gew% bis 12 Gew% der gesamten Lösung, oder Propylenglykol in der Menge von 5 Gew% bis 10 Gew% der gesamten Lösung, oder einer Mischung daraus in der Menge von 10 Gew% bis 15 Gew% der gesamten Lösung, umfaßt und
(c) Polyoxyl 35 Rizinusöl in der Menge von 5 Gew% bis 10 Gew% der gesamten Lösung.

25. Die Zusammensetzung von Anspruch 23, die folgendes umfaßt:
(a) eine Mischung aus Ritonavir in der Menge von 1 Gew% bis 30 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von 1 Gew% bis 50 Gew% der gesamten Lösung,
(b) ein pharmazeutisch verträgliches organisches Lösungsmittel, welches eine Mischung aus (1) Ölsäure in der Menge von 30 Gew% bis 88 Gew% der gesamten Lösung, und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

26. Die Zusammensetzung von Anspruch 25, die folgendes umfaßt:
(a) eine Mischung aus Ritonavir in der Menge von 5 Gew% bis 25 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von 5 Gew% bis 40 Gew% der gesamten Lösung,
(b) ein pharmazeutisch verträgliches organisches Lösungsmittel, welches eine Mischung aus (1) Ölsäure in der Menge von 40 Gew% bis 65 Gew% der gesamten Lösung, und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

27. Die Zusammensetzung von Anspruch 21, die folgendes umfaßt:
(a) eine Mischung aus Ritonavir in der Menge von ungefähr 5 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 30 Gew% der gesamten Lösung,
(b) ein pharmazeutisch verträgliches organisches Lösungsmittel, welches eine Mischung aus (1) Ölsäure in der Menge von ungefähr 45 Gew% der gesamten Lösung, und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

28. Die Zusammensetzung von Anspruch 21, die folgendes umfaßt:
(a) eine Mischung aus Ritonavir in der Menge von ungefähr 15 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 15 Gew% der gesamten Lösung,
(b) ein pharmazeutisch verträgliches organisches Lösungsmittel, welches eine Mischung aus (1) Ölsäure in der Menge von ungefähr 50 Gew% der gesamten Lösung, und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

29. Die Zusammensetzung von Anspruch 21, die folgendes umfaßt:
(a) eine Mischung aus Ritonavir in der Menge von ungefähr 15 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 5 Gew% der gesamten Lösung,
(b) ein pharmazeutisch verträgliches organisches Lösungsmittel, welches eine Mischung aus (1) Ölsäure in der Menge von ungefähr 60 Gew% der gesamten Lösung, und (2) Ethanol in der Menge von ungefähr 10 Gew% der gesamten Lösung umfaßt, und
(c) Polyoxyl 35 Rizinusöl in der Menge von ungefähr 10 Gew% der gesamten Lösung.

30. Die Zusammensetzung von Anspruch 21, die eine Mischung aus Ritonavir und (2S,3S,5S)-2-(2,6-dimethylphenoxyacetyl)amino-3-hydroxy-5-(2S-(1-tetrahydro-pyrimid-2-onyl)-3-methyl butanoyl) amino-1,6-diphenylhexan umfaßt, wobei das (m/m) Verhältnis von Ritonavir zu (2S, 3S, 5S)-2-(2,6-dimethylphenoxyacetyl)amino-3-hydroxy-5-(2S-(1-tetrahydro-pyrimid-2-onyl)-3-methyl butanoyl) amino-1,6-diphenylhexan von 1:16 bis 5:1 ist.

31. Die Zusammensetzung von Anspruch 30, die eine Mischung aus Ritonavir und (2S,3S,5S)-2-(2, 6-dimethylphenoxyacetyl) amino-3-hydroxy-5-(2S-(1-tetrahydro-pyrimid-2-onyl)-3-methyl butanoyl) amino-1,6-diphenylhexan umfaßt, wobei das (m/m) Verhältnis von Ritonavir zu (2S,3S,5S)-2-(2,6-dimethylphenoxyacetyl)amino-3-hydroxy-5-(2S-(1-tetrahydro-pyrimid-2-onyl)-3-methyl butanoyl) amino-1,6-diphenylhexan von 1:8 bis 3:1 ist.

32. Die Zusammensetzung von Anspruch 1, die folgendes umfaßt:
(a) eine Mischung aus Ritonavir in der Menge von 1 Gew% bis 30 Gew% der gesamten Lösung und eine andere HIV Protease-hemmende Verbindung in der Menge von 1 Gew% bis 50 Gew% der gesamten Lösung,
(b) ein pharmazeutisch verträgliches organisches Lösungsmittel, welches eine Mischung aus (1) Ölsäure in der Menge von 30 Gew% bis 88 Gew% der gesamten Lösung, und (2) Propylenglykol in der Menge von 5 Gew% bis 10 Gew% der gesamten Lösung umfaßt, und
(c) Polyoxyl 35 Rizinusöl in der Menge von ungefähr 10 Gew% der gesamten Lösung.

33. Die Zusammensetzung von Anspruch 32, die folgendes umfaßt:
(a) eine Mischung aus Ritonavir in der Menge von 5 Gew% bis 25 Gew% der gesamten Lösung und (2S,3S,5S)-2-(2,6-dimethylphenoxyacetyl) amino-3-hydroxy-5-(2S-(1-tetrahydropyrimid-2-onyl)-3-methyl butanoyl) amino-1,6-diphenylhexan in der Menge von 5 Gew% bis 40 Gew% der gesamten Lösung,
(b) ein pharmazeutisch verträgliches organisches Lösungsmittel, welches eine Mischung aus (1) Ölsäure in der Menge von 40 Gew% bis 65 Gew% der gesamten Lösung, und (2) Propylenglykol in der Menge von 6 Gew% bis 8 Gew% der gesamten Lösung umfaßt, und
(c) Polyoxyl 35 Rizinusöl in der Menge von ungefähr 10 Gew% der gesamten Lösung.

34. Die Zusammensetzung von Anspruch 32, die folgendes umfaßt:
(a) eine Mischung aus Ritonavir in der Menge von 1 Gew% bis 30 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von 1 Gew% bis 50 Gew% der gesamten Lösung,
(b) ein pharmazeutisch verträgliches organisches Lösungsmittel, welches eine Mischung aus (1) Ölsäure in der Menge von 30 Gew% bis 88 Gew% der gesamten Lösung, und (2) Propylenglykol in der Menge von 5 Gew% bis 10 Gew% der gesamten Lösung umfaßt, und
(c) Polyoxyl 35 Rizinusöl in der Menge von ungefähr 10 Gew% der gesamten Lösung.

35. Die Zusammensetzung von Anspruch 34, die folgendes umfaßt:
(a) eine Mischung aus Ritonavir in der Menge von 5 Gew% bis 25 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von 5 Gew% bis 40 Gew% der gesamten Lösung,
(b) ein pharmazeutisch verträgliches organisches Lösungsmittel, welches eine Mischung aus (1) Ölsäure in der Menge von 40 Gew% bis 65 Gew% der gesamten Lösung, und (2) Propylenglykol in der Menge von 6 Gew% bis 8 Gew% der gesamten Lösung umfaßt, und
(c) Polyoxyl 35 Rizinusöl in der Menge von ungefähr 10 Gew% der gesamten Lösung.

36. Die Zusammensetzung von Anspruch 35, die folgendes umfaßt:
(a) eine Mischung aus Ritonavir in der Menge von ungefähr 6,0 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 24 Gew% der gesamten Lösung,
(b) ein pharmazeutisch verträgliches organisches Lösungsmittel, welches eine Mischung aus (1) Ölsäure in der Menge von ungefähr 52,5 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

37. Die Zusammensetzung von Anspruch 35, die eine Lösung aus (a) einer Mischung aus Ritonavir in der Menge von ungefähr 5 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 25 Gew% der gesamten Lösung und (b) Polyoxyl 35 Rizinusöl in der Menge von ungefähr 10 Gew% der gesamten Lösung in einem pharmazeutisch verträglichen organischen Lösungsmittel umfaßt, welches eine Mischung aus (1) Ölsäure in der Menge von ungefähr 52,5 Gew% der gesamten Lösung, und (2) Propylenglykol in der Menge von ungefähr 7,5 Gew% der gesamten Lösung umfaßt.

38. Die Zusammensetzung von Anspruch 35, die folgendes umfaßt:
(a) eine Mischung aus Ritonavir in der Menge von ungefähr 8 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 24 Gew% der gesamten Lösung,
(b) ein pharmazeutisch verträgliches organisches Lösungsmittel, welches eine Mischung aus (1) Ölsäure in der Menge von ungefähr 50,5 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

39. Die Zusammensetzung von Anspruch 35, die folgendes umfaßt:
(a) eine Mischung aus Ritonavir in der Menge von ungefähr 8,25 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 22 Gew% der gesamten Lösung,
(b) ein pharmazeutisch verträgliches organisches Lösungsmittel, welches eine Mischung aus (1) Ölsäure in der Menge von ungefähr 52,25 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

40. Die Zusammensetzung von Anspruch 35, die folgendes umfaßt:
(a) eine Mischung aus Ritonavir in der Menge von ungefähr 5 Gew% der gesamten Lösung und (2S,3S,5S)-2-(2,6-dimethylphenoxyacetyl)amino-3-hydroxy-5-(2S-(1-tetrahydropyrimid-2-onyl)-3-methyl butanoyl) amino-1,6-diphenylhexan in der Menge von ungefähr 30 Gew% der gesamten Lösung,
(b) ein pharmazeutisch verträgliches organisches Lösungsmittel, welches eine Mischung aus (1) Ölsäure in der Menge von ungefähr 47,5 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

41. Die Zusammensetzung von Anspruch 35, die folgendes umfaßt:
(a) eine Mischung aus Ritonavir in der Menge von ungefähr 15 Gew% der gesamten Lösung und (2S,3S,5S)-2-(2,6-dimethylphenoxyacetyl)amino-3-hydroxy-5-(23-(1-tetrahydropyrimid-2-onyl)-3-methyl butanoyl) amino-1,6-diphenylhexan in der Menge von ungefähr 15 Gew% der gesamten Lösung,
(b) ein pharmazeutisch verträgliches organisches Lösungsmittel, welches eine Mischung aus (1) Ölsäure in der Menge von ungefähr 52,5 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

42. Die Zusammensetzung von Anspruch 35, die folgendes umfaßt:
(a) eine Mischung aus Ritonavir in der Menge von ungefähr 13 Gew% der gesamten Lösung und (2S,3S,5S)-2-(2,6-dimethylphenoxyacetyl) amino-3-hydroxy-5-(2S-(1-tetrahydropyrimid-2-onyl)-3-methyl butanoyl) amino-1,6-diphenylhexan in der Menge von ungefähr 17 Gew% der gesamten Lösung,
(b) ein pharmazeutisch verträgliches organisches Lösungsmittel, welches eine Mischung aus (1) Ölsäure in der Menge von ungefähr 52,5 Gew% der gesamten Lösung, und (2) Propylenglykol in der Menge von ungefähr 7,5 Gew% der gesamten Lösung umfaßt, und
(c) Polyoxyl 35 Rizinusöl in der Menge von ungefähr 10 Gew% der gesamten Lösung.

43. Die Zusammensetzung von Anspruch 35, die eine Mischung aus Ritonavir und (2S, 3S, 5S)-2-(2,6-dimethylphenoxyacetyl) amino-3-hydroxy-5-(2S-(1-tetrahydro-pyrimid-2-onyl)-3-methyl butanoyl) amino-1,6-diphenylhexan umfaßt, wobei das (m/m) Verhältnis von Ritonavir zu (2S,3S,5S)-2-(2,6-dimethylphenoxyacetyl) amino-3-hydroxy-5-(2S-(1-tetrahydro-pyrimid-2-onyl)-3-methyl butanoyl) amino-1,6-diphenylhexan von 1:16 bis 5:1 ist.

44. Die Zusammensetzung von Anspruch 41, die eine Mischung aus Ritonavir und (2S,3S,5S)-2-(2,6-dimethylphenoxyacetyl) amino-3-hydroxy-5-(2S-(1-tetrahydro-pyrimid-2-onyl)-3-methyl butanoyl) amino-1,6-diphenylhexan umfaßt, wobei das (m/m) Verhältnis von Ritonavir zu (2S,3S,5S)-2-(2,6-dimethylphenoxyacetyl)amino-3-hydroxy-5-(2S-(1-tetrahydro-pyrimid-2-onyl)-3-methyl butanoyl) amino-1,6-diphenylhexan von 1:8 bis 3:1 ist.

## Revendications

1. Composition pharmaceutique qui est une solution comprenant
(a) un composé inhibiteur de la protéase du VIH de formule 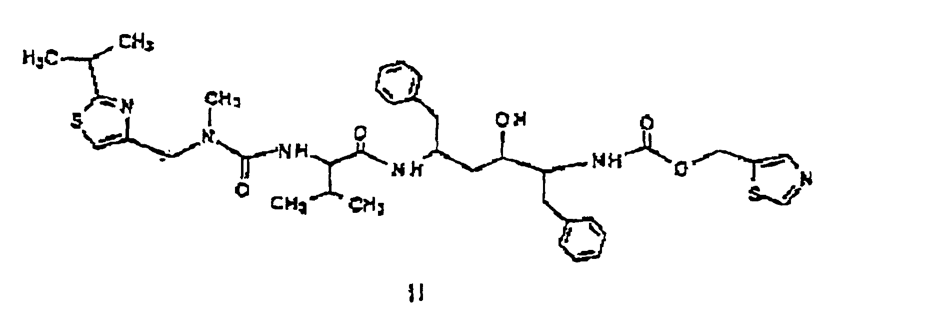 ou un de ses sels pharmaceutiquement acceptable, facultativement en association avec un autre composé inhibiteur du VIH, ou un de ses sels pharmaceutiquement acceptable, en une quantité totale de 1 % à 50 % en poids de la solution totale,
(b) un solvant organique pharmaceutiquement acceptable qui comprend (i) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 99 % en poids de la solution totale ou (ii) un mélange (1) d'un acide gras pharmaceutiquement acceptable choisi dans le groupe comprenant des acides carboxyliques saturés, mono-insaturés et di-insaturés en C₁₂ à C₁₈ qui sont des liquides à température ambiante dans un quantité de 30 % à 99 % en poids de la solution totale et
(2) d'un alcool pharmaceutiquement acceptable en une quantité de 0 % à 15 % en poids de la solution totale et
(c) un surfactant pharmaceutiquement acceptable en une quantité de 0 % à 40% en poids de la solution totale.

2. Composition de la revendication 1 dans laquelle la solution est encapsulée dans une capsule à enveloppe dure ou dans une capsule à enveloppe molle.

3. Composition de la revendication 1 dans laquelle le solvant comprend (1) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 40 % à 70 % en poids de la solution totale et (2) de l'éthanol ou du propylène glycol en une quantité de 1 % à 15 % en poids de la solution totale ou un mélange d'éthanol et de propylène glycol en une quantité de 1 % à 15 % en poids de la solution totale.

4. Composition de la revendication 1 dans laquelle le solvant comprend (1) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 40 % à 70 % en poids de la solution totale et (2) de l'éthanol en une quantité de 10 % à 12% en poids de la solution totale ou du propylène glycol en une quantité de 5 % à 10 % en poids de la solution totale ou un mélange d'éthanol et de propylène glycol en une quantité de 5 % à 15 % en poids de la solution totale.

5. Composition de la revendication 1 dans laquelle le solvant comprend (1) un acide oléique en une quantité de 40 % à 70 % en poids de la solution totale et (2) de l'éthanol en une quantité de 10 % à 12% en poids de la solution totale ou du propylène glycol en une quantité de 5 % à 10 % en poids de la solution totale ou un mélange d'éthanol et de propylène glycol en une quantité de 10 % à 15 % en poids de la solution totale.

6. composition de la revendication 1 dans laquelle le composé inhibiteur de la protéase du VIH est (2S,3S,5S)-5-(N-(N-((N-méthyl-N-((2-isopropyl-4-thiazolyl)méthyl)-amino) carbonyl)valinyl) amino)-2-(N-( (5-thiazolyl)méthoxycarbonyl)amino)-1,6-diphenyl-3-hydroxyhexane (ritonavir), ou un de ses sels pharmaceutiquement acceptable.

7. Composition de la revendication 1 dans laquelle le composé inhibiteur de la protéase du VIH en association avec le composé de formule II est choisi dans le groupe comprenant N-(2(R)-hydroxy-1(S)-indanyl)-2(R)-phénylméthyl-4 (S)-hydroxy-5-(1-(4-(3-pyridylméthyl)-2 (S)-N'-(t-butylcarboxamido)-pipérazinyl))-pentaneamide (indinavir) ;
N-tert-butyl-décahydro-2-(2 (R)-hydroxy-4-phényl-3(S)-))N-(2-quinolylcarbonyl)-L-asparaginyl)amino)butyl)-(4aS,8aS)-isocuinoline-3(S)-carboxamide (saquinavir) ; 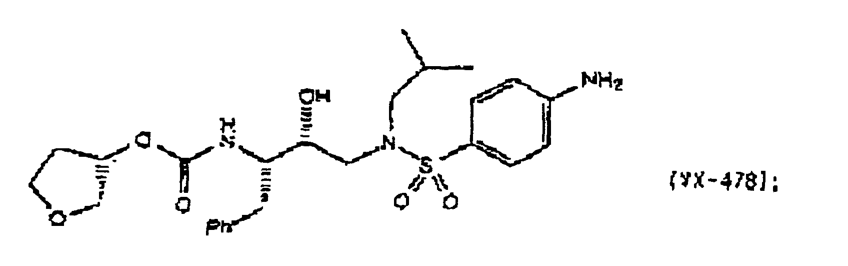 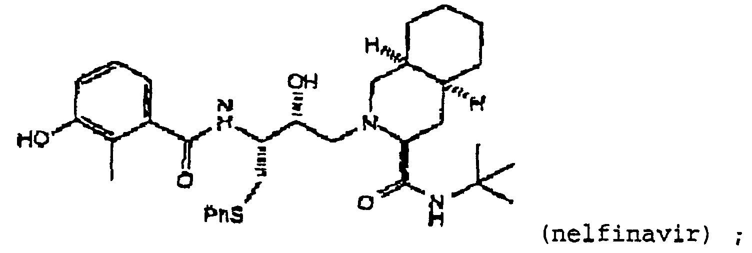 et un composé de formule 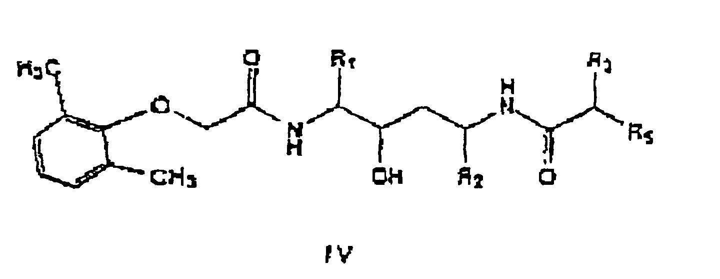 dans laquelle R₁ est un benzyle, R₂ est un benzyle ou un alkyle en C₁-C₆, R₃ est un alkyle en C₁ à C₆ et R₅ est 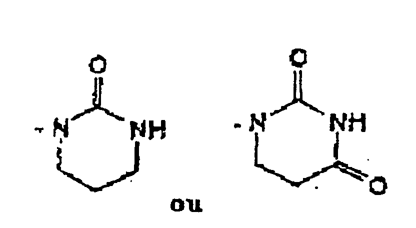 ou un sel pharmaceutiquement acceptable de l'un quelconque des composés ci-dessus.

8. Composition de la revendication 1 dans laquelle le composé inhibiteur de la protéase du VIH est ritonavir ou une association de ritonavir et d'un autre composé inhibiteur de la protéase du VIH.

9. Composition de la revendication 1 dans laquelle l'association de composés inhibiteurs de la protéase du VIH est la suivante :
ritonavir et (2S,3S,5S)-2-(2,6-diméthylphénoxyacétyl)-amino-3-hydroxy-5-(2S-(1-tétrahydro-pyrimid-2-onyl)-3-méthyl butanoyl)amino-1,6-diphénylhexane ;
ritonavir et indinavir ;
ritonavir et saquinavir ;
ritonavir et nelfinavir ou
ritonavir et VX-478.

10. Composition de la revendication 1 dans laquelle le composé inhibiteur de la protéase du VHI est ritonavir ou une association de ritonavir et de (2S,3S,5S)-2-(2,6-diméthylphenoxyacétyl)amino-3-hydroxy-5-(2S-(1-tétrahydro-pyrimid-2-onyl)-3-méthyl-butanoyl)amino-1,6-diphénylhexane.

11. Composition de la revendication 1 comprenant
(a) ritonavir en une quantité de 1% à 30% en poids de la solution totale.
(b) un solvant organique pharmaceutiquement acceptable qui comprend (i) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 99 % en poids de la solution totale ou (ii) un mélange de (1) un acide gras pharmaceutiquement acceptable choisi dans -le groupe comprenant des acides carboxyliques saturés, mono-insaturés et di-insaturés en C₁₂ à C₁₈ qui sont des liquides à température ambiante en une quantité de 30 % à 99 % en poids de la solution totale et (2) un alcool pharmaceutiquement acceptable en une quantité de 0 % à 15 % en poids de la solution totale et
(c) un surfactant pharmaceutiquement acceptable en une quantité de 0 % à 20% en poids de la solution totale.

12. Composition de la revendication 11 dans laquelle la solution est encapsulée dans une capsule à enveloppe molle.

13. Composition de la revendication 11 comprenant
(a) ritonavir en une quantité de 5% à 25% en poids de la solution totale.
(b) un solvant organique pharmaceutiquement acceptable qui comprend (i) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 70% en poids de la solution totale ou (ii) un mélange de (1) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 70% en poids de la solution totale et (2) un alcool pharmaceutiquement acceptable en une quantité de 6% à 12% en poids de la solution totale et
(c) un surfactant pharmaceutiquement acceptable en une quantité de 5% à 10% en poids de la solution totale.

14. Composition de la revendication 13 dans laquelle la solution est encapsulée dans une capsule à enveloppe molle.

15. Composition de la revendication 1 comprenant
(c) ritonavir en une quantité de 1 % à 30% en poids de la solution totale.
(b) un solvant organique pharmaceutiquement acceptable qui comprend (i) de l'acide oléique en une quantité de 30 % à 99 % en poids de la solution totale
ou (ii) un mélange (1) d'acide oléique en une quantité de 30% à 99% en poids de la solution totale et
(2) de l'éthanol en une quantité de 0 % à 12 % en poids de la solution totale ou du propylène glycol en une quantité de 0 % à 10 % en poids de la solution totale ou un mélange de ces deux substances en une quantité de 0 % à 15 % en poids de la solution totale et
(c) de l'huile de ricin polyoxyle 35 en une quantité de 0% à 20% en poids de la solution totale.

16. Composition de la revendication 15 comprenant
(a) ritonavir en une quantité de 5% à 25% en poids de la solution totale.
(b) un solvant organique pharmaceutiquement acceptable qui comprend (i) de l'acide oléique en une quantité de 30 % à 70 % en poids de la solution totale ou (ii) un mélange (1) d'acide oléique en une quantité de 30 % à 70 % en poids de la solution totale et
(2) de l'éthanol en une quantité de 10% à 12 % en poids de la solution totale ou du propylène glycol en une quantité de 5% à 10 % en poids de la solution totale ou un mélange de ces deux substances en une quantité de 10 % à 15 % en poids de la solution totale et
(c) de l'huile de ricin polyoxyle 35 en une quantité de 5% à 10% en poids de la solution totale.

17. Composition de la revendication 15 dans laquelle la solution est encapsulée dans une capsule à enveloppe molle.

18. Composition de la revendication 15 comprenant
(a) ritonavir en une quantité de environ 20 % en poids de la solution totale.
(b) un solvant organique pharmaceutiquement acceptable qui comprend un mélange (1) d'acide oléique en une quantité de 62 % à 64 % en poids de la solution totale et (2) de l'éthanol en une quantité de environ 10 % à environ 12 % en poids de la solution totale et
(c) de l'huile de ricin polyoxyle 35 en une quantité de environ 6% en poids de la solution totale.

19. Composition de la revendication 15 comprenant
(a) ritonavir en une quantité de environ 20 % en poids de la solution totale.
(b) un solvant organique pharmaceutiquement acceptable qui comprend un mélange (1) d'acide oléique en une quantité de environ 65 % en poids de la solution totale et (2) de l'éthanol en une quantité de environ 10 % en poids de la solution totale et
(c) de l'huile de ricin polyoxyle 35 en une quantité de environ 5% en poids de la solution totale.

20. Composition de la revendication 15 comprenant
(a) ritonavir en une quantité de environ 20 % en poids de la solution totale.
(b) un solvant organique pharmaceutiquement acceptable qui comprend un mélange (1) d'acide oléique en une quantité de environ 60% en poids de la solution totale et (2) de l'éthanol en une quantité de environ 10 % en poids de la solution totale et
(c) de l'huile de ricin polyoxyle 35 en une quantité de environ 10% en poids de la solution totale.

21. Composition de la revendication 1 comprenant
(a) un mélange de ritonavir en une quantité de 1 % à 30 % en poids de la solution totale et un autre inhibiteur de la protéase du VIH en une quantité de 1 % à 50 % en poids de la solution totale,
(b) un solvant organique pharmaceutiquement acceptable qui comprend (i) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 98% en poids de la solution totale ou (ii) un mélange de (1) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 98% en poids de la solution totale et (2) un alcool pharmaceutiquement acceptable en une quantité de 0 % à 15 % en poids de la solution totale et
(c) un surfactant pharmaceutiquement acceptable en une quantité de 0% à 20% en poids de la solution totale.

22. Composition de la revendication 21 comprenant
(a) un mélange de ritonavir en une quantité de 5% à 25% en poids de la solution totale et un autre inhibiteur de la protéase du VIH en une quantité de 5% à 40% en poids de la solution totale,
(b) un solvant organique pharmaceutiquement acceptable qui comprend (i) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30% à 70% en poids de la solution totale ou (ii) un mélange de (1) un acide gras pharmaceutiquement acceptable choisi dans le groupe comprenant des acides carboxyliques saturés, mono-insaturés et di-insaturés en C₁₂ à C₁₈ qui sont des liquides à température ambiante en une quantité de 30 % à 70% en poids de la solution totale et (2) un alcool pharmaceutiquement acceptable en une quantité de 6% à 12% en poids de la solution totale et
(c) un surfactant pharmaceutiquement acceptable en une quantité de 5% à 10% en poids de la solution totale.

23. Composition de la revendication 21 comprenant
(a) un mélange de ritonavir en une quantité de 1% à 30% en poids de la solution totale et un autre inhibiteur de la protéase du VIH en une quantité de 1% à 50% en poids de la solution totale,
(b) un solvant organique pharmaceutiquement acceptable qui comprend (i) de l'acide oléique en une quantité de 30 % à 98 % en poids de la solution totale ou (ii) un mélange (1) d'acide oléique en une quantité de 30 % à 98 % en poids de la solution totale et (2) d'éthanol en une quantité de environ 0 % à 12 % en poids de la solution totale ou du propylène glycol en une quantité de 0 % à 10 % en poids de la solution totale ou un mélange de ces deux substances en une quantité de 0 % à 15 % en poids de la solution totale et
(c) de l'huile de ricin polyoxyle 35 en une quantité de 0% à 20% en poids de la solution totale.

24. Composition de la revendication 23 comprenant
(a) un mélange de ritonavir en une quantité de 5% à 25% en poids de la solution totale et un autre inhibiteur de la protéase du VIH en une quantité de 5% à 40% en poids de la solution totale,
(b) un solvant organique pharmaceutiquement acceptable qui comprend (i) de l'acide oléique en une quantité de 30 % à 70 % en poids de la solution totale ou (ii) un mélange (1) d'acide oléique en une quantité de 30 % à 70 % en poids de la solution totale et (2) de l'éthanol en une quantité de 10% à 12 % en poids de la solution totale ou du propylène glycol en une quantité de 5% à 10 % en poids de la solution totale ou un mélange de ces deux substances en une quantité de 10% à 15 % en poids de la solution totale et
(c) de l'huile de ricin polyoxyle 35 en une quantité de 5% à 10% en poids de la solution totale.

25. Composition de la revendication 23 comprenant
(a) un mélange de ritonavir en une quantité de 1 % à 30 % en poids de la solution totale et de (2S,3S,5S)-2-(2,6-diméthylphénoxyacétyl)amino-3-hydroxy-5-(2S-(1-tétrahydro-pyrimid-2-onyl)-3-méthyl butanoyl)amino-1,6-diphénylhexane en une quantité de 1 % à 50 % en poids de la solution totale,
(b) un solvant organique pharmaceutiquement acceptable qui comprend un mélange (1) d'acide oléique en une quantité de 30 % à 88 % en poids de la solution totale et (2) de l'éthanol en une quantité de environ 10 % en poids de la solution totale et
(c) de l'huile de ricin polyoxyle 35 en une quantité de environ 10% en poids de la solution totale.

26. Composition de la revendication 25 comprenant
(a) un mélange de ritonavir en une quantité de 5 % à 25 % en poids de la solution totale et de (2S, 3S, 5S)-2-(2,6-diméthylphénoxyacétyl)amino-3-hydroxy-5-(2S-(1-tétrahydro-pyrimid-2-onyl)-3-méthyl butanoyl) amino-1,6-diphénylhexane en une quantité de 5 % à 40 % en poids de la solution totale,
(b) un solvant organique pharmaceuciquement acceptable qui comprend un mélange (1) d'acide oléique en une quantité de 40 % à 65 % en poids de la solution totale et (2) de l'éthanol en une quantité de environ 10 % en poids de la solution totale et
(c) de l'huile de ricin polyoxyle 35 en une quantité de environ 10% en poids de la solution totale.

27. Composition de la revendication 21 comprenant
(a) un mélange de ritonavir en une quantité de environ 5% en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 30 % en poids de la solution totale,
(b) un solvant organique pharmaceutiquement acceptable qui comprend un mélange (1) d'acide oléique en une quantité de environ 45 % en poids de la solution totale et (2) de l'éthanol en une quantité de environ 10 % en poids de la solution totale et
(c) de l'huile de ricin polyoxyle 35 en une quantité de environ 10% en poids de la solution totale.

28. Composition de la revendication 21 comprenant
(a) un mélange de ritonavir en une quantité de environ 15 % en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 15 % en poids de la solution totale,
(b) un solvant organique pharmaceutiquement acceptable qui comprend un mélange (1) d'acide oléique en une quantité de environ 50% en poids de la solution totale et (2) de l'éthanol en une quantité de environ 10 % en poids de la solution totale et
(c) de l'huile de ricin polyoxyle 35 en une quantité de environ 10% en poids de la solution totale.

29. Composition de la revendication 21 comprenant
(a) un mélange de ritonavir en une quantité de environ 15 % en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 5 % en poids de la solution totale,
(b) un solvant organique pharmaceutiquement acceptable qui comprend un mélange (1) d'acide oléique en une quantité de environ 60 % en poids de la solution totale et (2) de l'éthanol en une quantité de environ 10 % en poids de la solution totale et
(c) de l'huile de ricin polyoxyle 35 en une quantité de environ 10% en poids de la solution totale.

30. Composition de la revendication 21 comprenant un mélange de ritonavir et de (2S,3S,5S)-2=(2,6-diméthylphénoxyacétyl)amino-3-hydroxy-5-(2S-(1-tétrahydro-pyrimid-2-onyl)-3-méthyl-butanoyl) amine-1, 6-diphénylhexane, le ratio (p/p) de ritonavir sur (2S,3S,5S)-2-(2,6-diméthylphénoxyacétyl) amino-3-hydroxy-5-(2S-(1-tétrahydro-pyrimid-2-onyl)-3-méthyl butanoyl) amino-1,6-diphénylhexane étant de 1:16 à 5:1.

31. Composition de la revendication 30 comprenant un mélange de ritonavir et de (2S,3S,5S)-2=(2,6-diméthylphénoxyacétyl)amino-3-hydroxy-5-(2S-(1-tétrahydro-pyrimid-2-onyl)-3-méthyl-butanoyl) amino-1,6-diphénylhexane, le ratio (p/p) de ritonavir sur (2S,3S,5S)-2-(2,6-diméthylphénoxyacétyl) amino-3-hydroxy-5-(2S-(1-tétrahydro-pyrimid-2-onyl)-3-méthyl butanoyl) amino-1,6-diphénylhexane étant de 1:8 à 3:1.

32. Composition de la revendication 1 comprenant
(a) un mélange de ritonavir en une quantité de 1 % à 30 % en poids de la solution totale et d'un autre inhibiteur de la protéase du VIH en une quantité de 1 % à 50 % en poids de la solution totale.
(b) un solvant organique pharmaceutiquement acceptable qui comprend un mélange (1) d'acide oléique en une quantité de 30 % à 88 % en poids de la solution totale et (2) du propylène glycol en une quantité de 5 % à 10 % en poids de la solution totale et
(c) de l'huile de ricin polyoxyle 35 en une quantité de environ 10% en poids de la solution totale.

33. Composition de la revendication 32 comprenant
(a) un mélange de ritonavir en une quantité de 5 % à 25 % en poids de la solution totale et de (2S,3S,5S)-2-(2,6-diméthylphénoxyacétyl)amino-3-hydroxy-5-(2S-(1-tétrahydro-pyrimid-2-onyl)-3-méthyl butanoyl)amino-1,6-diphénylhexane en une quantité de 5 % à 40 % en poids de la solution totale,
(b) un solvant organique pharmaceutiquement acceptable qui comprend un mélange (1) d'acide oléique en une quantité de 40 % à 65 % en poids de la solution totale et (2) du propylène glycol en une quantité de 6 % à 8 % en poids de la solution totale et
(c) de l'huile de ricin polyoxyle 35 en une quantité de environ 10% en poids de la solution totale.

34. Composition de la revendication 32 comprenant
(a) un mélange de ritonavir en une quantité de 1 % à 30 % en poids de la solution totale et de (2S, 3S, 5S)-2-(2, 6-diméthylphénoxyacétyl)amino-3-hydroxy-5-(2S-(1-tétrahydro-pyrimid-2-onyl)-3-méthyl butanoyl)amino-1,6-diphénylhexane en une quantité de 1 % à 50 % en poids de la solution totale,
(b) ) un solvant organique pharmaceutiquement acceptable qui comprend un mélange (1) d'acide oléique en une quantité de 30 % à 88 % en poids de la solution totale et (2) du propylène glycol en une quantité de 5 % à 10 % en poids de la solution totale et
(c) de l'huile de ricin polyoxyle 35 en une quantité de environ 10% en poids de la solution totale.

35. Composition de la revendication 34 comprenant
(a) un mélange de ritonavir en une quantité de 5 % à 25 % en poids de la solution totale et de (2S,3S,5S)-2-(2,6-diméthylphénoxyacétyl)amino-3-hydroxy-5-(2S-(1-tétrahydro-pyrimid-2-onyl)-3-méthyl butanoyl)amino-1,6-diphénylhexane en une quantité de 5 % à 40 % en poids de la solution totale,
(b) un solvant organique pharmaceutiquement acceptable qui comprend un mélange (1) d'acide oléique en une quantité de 40 % à 65 % en poids de la solution totale et (2) du propylène glycol en une quantité de 6 % à 8 % en poids de la solution totale et
(c) de l'huile de ricin polyoxyle 35 en une quantité de environ 10% en poids de la solution totale.

36. Composition de la revendication 35 comprenant
(a) un mélange de ritonavir en une quantité de environ 6,0 % en poids de la solution totale et de (2S,3S,5S)-2-(2, 6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 24 % en poids de la solution totale,
(b) un solvant organique pharmaceutiquement acceptable qui comprend un mélange (1) d'acide oléique en une quantité de environ 52,5 % en poids de la solution totale et (2) du propylène glycol en une quantité de environ 7,5 % en poids de la solution totale et
(c) de l'huile de ricin polyoxyle 35 en une quantité de environ 10% en poids de la solution totale.

37. Composition de la revendication 35 comprenant une solution (a) d'un mélange de ritonavir en une quantité de environ 5 % en poids de la solution totale et de (2S,3S,5S)-2-(2,6-diméthylphénoxyacétyl) amino-3-hydroxy-5-(2S-(1-tétrahydro-pyrimid-2-onyl)-3-méthyl butanoyl) amino-1,6-diphénylhexane en une quantité de environ 25 % en poids de la solution totale et (b) de l'huile de ricin polyoxyle 35 en une quantité de environ 10 % en poids de la solution totale, dans un solvant organique pharmaceutiquement acceptable qui comprend un mélange (1) d'acide oléique en une quantité de environ 52,5 % en poids de la solution totale et (2) du propylène glycol en une quantité de environ 7,5 % en poids de la solution totale.

38. Composition de la revendication 35 comprenant
(a) un mélange de ritonavir en une quantité de environ 8% en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 24 % en poids de la solution totale,
(b) un solvant organique pharmaceutiquement acceptable qui comprend un mélange (1) d'acide oléique en une quantité de environ 50,5 % en poids de la solution totale et (2) du propylène glycol en une quantité de environ 7,5 % en poids de la solution totale et
(c) de l'huile de ricin polyoxyle 35 en une quantité de environ 10% en poids de la solution totale.

39. Composition de la revendication 35 comprenant
(a) un mélange de ritonavir en une quantité de environ 8,25 % en poids de la solution totale et de (2S, 3S, 5S)-2-(2-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 22 % en poids de la solution totale,
(b) un solvant organique pharmaceutiquement acceptable qui comprend un mélange (1) d'acide oléique en une quantité de environ 52,25% en poids de la solution totale et (2) du propylène glycol en une quantité de environ 7,5 % en poids de la solution totale et
(c) de l'huile de ricin polyoxyle 35 en une quantité de environ 10% en poids de la solution totale.

40. Composition de la revendication 35 comprenant
(a) un mélange de ritonavir en une quantité de environ 5 % en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl) amino-1,6-diphénylhexane en une quantité de environ 30 % en poids de la solution totale,
(b) un solvant organique pharmaceutiquement acceptable qui comprend un mélange (1) d'acide oléique en une quantité de environ 47,5 % en poids de la solution totale et (2) du propylène glycol en une quantité de environ 7,5 % en poids de la solution totale et
(c) de l'huile de ricin polyoxyle 35 en une quantité de environ 10% en poids de la solution totale.

41. Composition de la revendication 35 comprenant
(a) un mélange de ritonavir en une quantité de environ 15 % en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 15 % en poids de la solution totale,
(b) un solvant organique pharmaceutiquement acceptable qui comprend un mélange (1) d'acide oléique en une quantité de environ 52,5 % en poids de la solution totale et (2) du propylène glycol en une quantité de environ 7,5 % en poids de la solution totale et
(c) de l'huile de ricin polyoxyle 35 en une quantité de environ 10% en poids de la solution totale.

42. Composition de la revendication 35 comprenant
(a) un mélange de ritonavir en une quantité de environ 13 % en poids de la solution totale et de (2S,3S,5S)-2-(2,6-diméthylphénoxyacétyl)amino-3-hydroxy-5-(2S-(1-tétrahydro-pyrimid-2-onyl)-3-méthylbutanoyl)amino-1,6-diphénylhexane en une quantité de environ 17 % en poids de la solution totale,
(b) un solvant organique pharmaceutiquement acceptable qui comprend un mélange (1) d'acide oléique en une quantité de environ 52,5 % en poids de la solution totale et (2) du propylène glycol en une quantité de environ 7,5 % en poids de la solution totale et
(c) de l'huile de ricin polyoxyle 35 en une quantité de environ 10% en poids de la solution totale.

43. Composition de la revendication 35 comprenant un mélange de ritonavir et de (2S,3S,5S)-2=(2,6-diméchylphénoxyacétyl)amino-3-hydroxy-5-(2S-(1-tétrahydro-pyrimid-2-onyl)-3-méthyl-butanoyl)amino-1,6-diphénylhexane, le ratio (p/p) de riconavir sur (2S, 3S, 5S)-2-(2,6-diméthylphénoxyacétyl) amino-3-hydroxy-5-(2S-(1-tétrahydro-pyrimid-2-onyl)-3-méthyl butanoyl) amino-1,6-diphénylhexane étant de 1:16 à 5:1.

44. Composition de la revendication 41 comprenant un mélange de ritonavir et de (2S,3S,5S)-2=(2,6-diméthylphénoxyacétyl)amino-3-hydroxy-5-(2S-(1-tétrahydro-pyrimid-2-onyl)-3-méthyl-butanoyl)amino-1,6-diphénylhexane, le ratio (p/p) de ritonavir sur (2S,3S,5S)-2-(2,6-diméthylphénoxyacétyl) amino-3-hydroxy-5-(2S-(1-tétrahydro-pyrimid-2-onyl)-3-méthyl butanoyl) amino-1,6-diphénylhexane étant de 1:8 à 3:1.
